# EUROPÄISCHE PATENTSCHRIFT

(11) **EP 1 824 457 B1**
(45) Veröffentlichungstag und Bekanntmachung des Hinweises auf die Patenterteilung: **17.02.2016**
(21) Anmeldenummer: 05807707.4
(22) Anmeldetag: 05.11.2005
(51) Int. Cl.: A61K 9/16, A61K 31/7088

(54) **MULTIPARTIKULÄRE ARZNEIFORM, ENTHALTEND MUCOADHAESIV FORMULIERTE NUKLEINSÄURE-WIRKSTOFFE, SOWIE EIN VERFAHREN ZUR HERSTELLUNG DER ARZNEIFORM**
MULTIPARTICULATE FORM OF ADMINISTRATION, COMPRISING NUCLEIC ACID-CONTAINING MUCOADHESIVE ACTIVE INGREDIENTS, AND METHOD FOR PRODUCING SAID FORM OF ADMINISTRATION
FORME GALENIQUE MULTIPARTICULAIRE RENFERMANT DES PRINCIPES ACTIFS CONTENANT UN ACIDE NUCLEIQUE A FORMULATION MUCOADHESIVE, AINSI QUE PROCEDE POUR PRODUIRE CETTE FORME GALENIQUE

(30) Priorität: 10.12.2004 DE 102004059792
(43) Veröffentlichungstag der Anmeldung: 29.08.2007
(73) Patentinhaber: Evonik Röhm GmbH, 64293 Darmstadt (DE)
(72) Erfinder: LIZIO, Rosario, 64380 Rossdorf (DE); PETEREIT, Hans-Ulrich, 64291 Darmstadt (DE); TRUPTI, Dave, 64673 Zwingenberg (DE); GOTTSCHALK, Michael, 64372 Ober-Ramstadt (DE)
(86) Internationale Anmeldenummer: PCT/EP2005/011864
(87) Internationale Veröffentlichungsnummer: WO 2006/061069

(56) Entgegenhaltungen:
- EP-A- 0 514 008
- EP-A- 1 203 590
- US-A1- 2003 166 783
- US-B1- 6 465 626
- HEJAZI R ET AL: "Chitosan-based gastrointestinal delivery systems" JOURNAL OF CONTROLLED RELEASE, ELSEVIER, AMSTERDAM, NL, Bd. 89, Nr. 2, 29. April 2003 (2003-04-29), Seiten 151-165, XP004421352 ISSN: 0168-3659
- ROY K ET AL: "Oral gene delivery with chitosan-DNA nanoparticles generates immunologic protection in a murine model of peanut allergy" NATURE MEDICINE, NATURE AMERICA, NEW YORK, US, Bd. 5, Nr. 4, April 1999 (1999-04), Seiten 387-391, XP002148464 ISSN: 1078-8956

## Beschreibung

Die Erfindung betrifft eine multipartikuläre Arzneiform, enthaltend mucoadhaesiv formulierte Nukleinsäure-Wirkstoffe, sowie ein Verfahren zur Herstellung der Arzneiform.

### Stand der Technik

WO 02/64148 beschreibt Formulierungen enthaltend ein Mucopolysaccharid und ein Verfahren zu ihrer Herstellung. Dabei wird ein Mucopolysaccharid, z. B. Heparin, zusammen mit einem Adsorptionsverstärker, z. B. einem Chitosan, formuliert und anschließend mit einem darmsaftlöslichen Überzug ausgestattet, so daß der Wirkstoff in den mittleren oder unteren Abschnitten des Dünndarms freigesetzt werden kann. Als darmsaftlösliche Überzüge kommen z. B. anionische Acrylcopolymere vom Typ EUDRAGIT® L, S, L100-55 in Betracht. Die Formulierungen können Kapseln, Tabletten und Granulate umfassen.

Die Telomerase ist ein Enzym, das bei Zellteilungen für die DNA-Verdopplung insbesondere-im Bereich der Chromosomen-Enden beiträgt. Das Enzym ist daher wichtig für die Aufrechterhaltung einer intakten Chromosomenstruktur. Die Telomerase-Aktivität ist in den meisten adulten Körperzellen reprimiert, lediglich in Keimzellen, aber auch in vielen Tumorzelltypen wird eine erhöhte Telomeraseaktivität beobachtet. Es wird vermutet, daß die Telomerase eine wichtige Rolle bei der molekularen Kontrolle des normalen Lebenszyklus von Zellen, bis hin zu ihrem genetisch vorprogrammierten Zelltod spielt. Die von normalen Zellen abweichend hohe Telomerase-Aktivität in Tumorzellen wird als Anzeichen dafür gedeutet, dass die normale Zellteilungskontrolle abhanden gekommen ist. Die Telomerase bzw. die damit verbundenen Genstrukturen werden als ein Ansatzpunkt zur genetischen Therapie von Tumorzellen gesehen.

WO 99/38964 beschreibt Nukleinsäuren für die Gentherapie, die insbesondere einen Telomerase Gen-Promotor enthalten. Diese DNA kann mit heterologen Genen wie z. B. Cytotoxin-kodierenden Genen gekoppelt werden. Das Nukleinsäure-Konstrukt kann als Wirkstoff zur Transfektion von Tumorzellen mit erhöhter Telomerase-Aktivität eingesetzt werden. Man verspricht sich davon eine Hemmung der Tumorzellteilung bis hin zur gezielten Abtötung dieser Zellen. Die Möglichkeit einer oralen Verabreichung der in der WO 99/38964 beschriebenen Wirkstofftypen bzw. daraus abgeleiteter Arzneiformen wird erwähnt.

Hejazi et al. (2003) beschreibt in J. Contr. Rel., Vol 89 S.151-165, "Chitosan-based gastrointestinal delivery systems" eine orale Verabreichungsform für DNA komplexiert mit Chitosan. US 6,465,626 offenbart Mikropartikel aus Chitosan und Gelatine, die Nukleinsäure beinhalten können.

Roy et al. (1999) beschreibt in Nature Medicine, Vol.5, Nr.4, S. 387- 391, "Oral gene delivery with chitosan-DNA nanoparticles generates immunologic protection in murine model of peanut allergy*"* eine orale Verabreichung eines DNA-Wirkstoffs in Mäusen. Das auf einer Plasmid-DNA vorhandene dominante Erdnuss-Allergen-Gen (pCMVArah2) wurden zusammen mit Chitosan mit einem Mw von ca. 390.000 mittels Komplex-Acervation zu Nanopartikeln einer Größe im Bereich von 100 bis 200 nm formuliert. Diese Nanopartikel wurden AKR/J-Mäusen oral verabreicht, wobei eine transduzierte Genexpression in den intestinalen Epithelzellen nachgewiesen werden konnte. Die so behandelten Mäuse produzierten Allergen-spezifische sekretorische IgA- und Serum IgG2a-Antikörper und zeigten im Vergleich zu einer Kontrollgruppe eine reduzierte, Allergen induzierte Anaphylaxe.

Leong et al. (1998) beschreibt in Journal of Controlled Release 53, S. 183 - 193 "DNA-polycation nanospheres as non-viral gene delivery vehicles*"* Gentransfer-Vehikel, die bei BALB/c-Mäusen in-vivo eine Fremdgenexpression bewirken. Die Nanospheren wurden als DNA-Komplexe mit Gelatine oder Chitosan mit einer Größe im bereich von 200 bis 700 nm hergestellt.

WO 02/094983 beschreibt Formulierungen aus Nukleinsäuren, Antikörpern mit Spezifität für DNA und diesbezüglichen kationischen Makromolekül-Komplexen. Die Formulierung erfolgt in Form von Nanopartikeln, wobei gesteigerte Transfektionsraten sowohl in-vitro als auch in-vivo nachgewiesen werden. Formulierungen für die orale Verabreichung mit verzögerter Wirkstofffreisetzung werden erwähnt.

WO 03/007913 beschreibt orale multipartikuläre Arzneiformen, die den Wirkstoff in Form einer Vielzahl sogenannter "Patches" enthalten. Ein "Patch" ist ein diskusartiges Objekt aus biokompatiblen Material mit einem Durchmesser von 500 µm bis 5 mm und einer Höhe von 100 bis 1000 µm. Das Patch besteht aus zwei Schichten bzw. Seiten, einer für Wasser oder Körperflüssigkeiten nur gering permeablen Seite, z. B. aus Ethylcellulose, und einer zweiten Seite, die den Wirkstoff, z. B. ein Protein, ein Polysaccharid oder ein kleines Molekül, enthält, der in Mischung mit mucoadhaesiven Polymeren, z. B. Chitosan, CMC, Polyacrylsäure oder Pektin, vorliegen kann. Die Patches können zu einer Tablette verpresst werden oder auch in eine Kapsel gefüllt werden, die zusätzlich mit einem darmsaftlöslichen Überzug ausgestattet wird. Die Wirkstoffpräparationen können auch zusätzlich mit sogenannten Enhancern, wie Fettsäuren, Fettalkoholen, Estern, oberflächenaktiven Substanzen und Proteaseinhibitoren kombiniert werden. Am Wirkort, z. B. im einem bestimmten Darmabschnitt, löst sich die Kapsel auf und gibt die "Patches" frei. Die freigesetzten "Patches" können mit ihrer mucoadhaesiven Seite an der Darmmucosa anhaften und dort den Wirkstoff verzögert und auf die Darmmucosa gerichtet abgeben. Die zweite, nur gering permeable Seite der "Patches" soll dem Wirkstoff einen gewissen Schutz gegenüber chemischer oder enzymatischer Inaktivierung von der Seite des Darmlumens her bieten und auch verhindern, daß der Wirkstoff nach dieser Seite hin entweicht.

WO 03/092732 beschreibt pH-sensitive Polymere, basierend auf anionischen (Meth)acrylatcopolymeren mit vergleichsweise niedrigem Molekulargewicht Mw von 1000 bis 50.000. Die pH-sensitiven Polymere eigenen sich u.a. auch zur Komplexierung von Nukleinsäuren. Die pH-sensitiven Polymere weisen im Bereich von pH 7,0 oder leicht darüber keine oder nur in hohen Konzentrationen cytotoxische Eigenschaften auf, wirken aber unterhalb von pH 6,5 bereits in geringer Konzentration in vivo zelltoxisch, bzw. hämolytisch bzw. membranolytisch.

### Aufgabe und Lösung

Die WO 99/38964 beschreibt Nukleinsäuren und Vektoren, betreffend das menschliche Telomerase-Gen bzw. den Promoter dieses Gens. Die dort beschriebenen Nukleinsäuren können als potentielle Wirkstoffe für die Gentherapie von Tumorzellen gelten. Die orale Verabreichung der in der WO 99/38964 beschriebenen Wirkstofftypen wird lediglich sehr allgemein angeregt. Es besteht ein Bedarf an Formulierungsvorschlägen, die einen Fachmann in die Lage versetzen, Wirkstoffe dieses Typs so zum Wirkort zu transportieren, dass eine vorzeitige Inaktivierung, insbesondere durch Nukleasen, ausbleibt und ein ausreichender Anteil des Wirkstoffs zur Transfektion der Zielzellen gelangt. Auch die eingangs erwähnte WO 02/094983, die Antikörper-DNA-Konjugat-Komplexe in Nanopartikeln beschreibt, gibt zur Formulierung von oralen Arzneiformen nur recht allgemeine Hinweise.

Die WO 03/007913 beschreibt eine Lösungsmöglichkeit zur Bereitstellung von oralen Arzneiformen, die im Darmlumen freigesetzt werden und dort zur Wirkung gelangen sollen. Ein Nachteil dieser Lösung kann unter anderem in dem aufwendigen Aufbau und der Herstellung der zweischichtigen "Patch"-Strukturen gesehen werden. Ungünstig erscheint insbesondere die Bereitstellung der Arzneiform als Kapsel mit einem magensaftresistenten, darmsaftlöslichen Überzug. Bei einer Größe von deutlich über 2,5 mm ist eine ungenügende therapeutische Reproduzierbarkeit zu befürchten. Die Passagezeit der Kapsel durch den Magen kann stark variieren In jedem Fall ist mit einem verzögerten Wirkungseintritt zu rechnen. Außerdem kann sich die Kapsel schon nach einem teilweisen Auflösen des Überzugs ihrerseits schnell oder langsam auflösen. Beide Prinzipien, Überzug und Kapsel, überlagern sich hier in ungünstiger Weise, so daß mit einer insgesamt unkontrollierten Freisetzung der "Patches" zu rechnen ist. Die Kapsel kann in einem Zustand, wo sie zumindest bereits teilweise den Darmsäften zugänglich ist, je nach aktuellem Darminhalt oder Darmperistaltik intakt bleiben oder auch mechanisch weitgehend zerstört werden. Es kann einerseits zu einer schlagartigen Freisetzung großer Mengen an "Patches" kommen oder andererseits auch zu einer ungewünscht verzögerten Freisetzung, je nach Zerfall oder mechanischer Belastung des zunächst überzogenen Kapselgebildes. Eine insgesamt besser zu kontrollierende Wirkstoffabgabe wäre daher wünschenswert.

Die vorliegende Erfindung betrifft oral verabreichbare Arzneiformen für Nukleinsäure-Wirkstoffe, insbesondere für die Zwecke der Gen-Therapie. Ein generelles Problem ist dabei, den Wirkstoff in einer Form zu formulieren, die die Transfektion von lebenden Zellen am Wirkort begünstigt und zugleich sicherzustellen, dass der Wirkstoff oder zumindest eine ausreichende Menge in transfektionsfähiger Form zum Wirkort gelangt. Es wurde als eine der Aufgaben der Erfindung gesehen, eine Arzneiform bereitzustellen, die sich zur gezielten und effektiven Freisetzung von Nukleinsäure-Wirkstoffen eignet. Die Arzneiform soll eine hohe Dosiersicherheit bieten und sich nach schneller Magenpassage gut im Darmlumen verteilen. Der enthaltene Nukleinsäure-Wirkstoff soll dabei weitgehend gegenüber physikalischer, chemischer oder nukleolytischer Inaktivierung geschützt sein und am definierten Wirkort so freizusetzen sein, dass ein hoher Anteil des Wirkstoffs von Körper aufgenommen werden kann. Der Freisetzungsort soll je nach therapeutischem Ziel variabel und zuverlässig einstellbar sein. Die Arzneiform soll neben dem DNA-Wirkstoff nur pharmakologisch unbedenkliche, nicht toxische Bestandteile aufweisen, so dass im primären Ansatz auch bei häufiger oder regelmäßiger Einnahme der Arzneiform keine unerwünschten Nebenwirkungen zu erwarten sind.

Die Aufgabe wird gelöst durch eine
Orale multipartikuläre Arzneiform, enthaltend Pellets mit einem mittleren Durchmesser im Bereich von 50 bis 2500 µm, die aufgebaut sind aus
a) einer inneren Matrix-Schicht, enthaltend Nanopartikel, die einen Nukleinsäure-Wirkstoff enthalten, und in eine Matrix aus einem Polymeren mit mucoadhaesiver Wirkung eingebettet sind, wobei die Matrix optional weitere pharmazeutisch übliche Hilfsstoffe enthalten kann,
b) einem äußeren verfilmten Überzug, bestehend im wesentlichen aus einem anionischen Polymeren oder Copolymeren, das optional mit pharmazeutisch üblichen Hilfsstoffen, insbesondere Weichmachern formuliert sein kann,
   dadurch gekennzeichnet, daß
die multipartikuläre Arzneiform so formuliert ist, daß die enthaltenen Pellets im pH-Bereich des Magens freigesetzt werden, der äußere Überzug durch die Wahl des anionischen Polymeren oder Copolymeren bzw. seiner Formulierung mit Hilfsstoffen und seiner Schichtdicke so eingestellt ist, daß sich dieser in pH-Bereichen von 4,0 bis 8,0 im Darm innerhalb 15 bis 60 min auflöst, so daß die wirkstoffhaltige, mucoadhaesive Matrix-Schicht frei wird, an die Darmmucosa binden und dort den Wirkstoff freisetzen kann, wobei das Polymere mit mucoadhaesiver Wirkung so gewählt ist, daß es in einem Bereich +/- 0,5 pH-Einheiten bezogen auf den pH-Wert, bei dem sich der äußere Überzug aufzulösen beginnt, eine mucoadhaesive Wirkung von mindestens η_{b} = 150 bis 1000 mPa·s und eine Wasseraufnahme von 10 bis 750 % in 15 min aufweist und der Wirkstoffanteil der Nanopartikel in der Matrixschicht maximal 40 Gew.-% des Gehaltes am Polymeren mit mucoadhaesiver Wirkung beträgt.

### Ausführung der Erfindung

Die Erfindung betrifft eine orale multipartikuläre Arzneiform, insbesondere in Form einer Tablette, Mini-Tablette, in Kapseln verfüllter Pellets, Sachets oder Trockensäfte, enthaltend Pellets mit einer mittleren Größe, bzw. mittlerem Durchmesser im Bereich von 50 bis 2500, bevorzugt von 100 bis 1000 µm, die aufgebaut sind aus
a) einer inneren Matrix-Schicht, enthaltend Nanopartikel, die einen Nukleinsäure-Wirkstoff enthalten, und in eine Matrix aus einem Polymeren mit mucoadhaesiver Wirkung eingebettet sind, wobei die Matrix optional weitere pharmazeutisch übliche Hilfsstoffe enthalten kann,
b) einem äußeren verfilmten Überzug, bestehend im wesentlichen aus einem anionischen Polymeren oder Copolymeren, das optional mit pharmazeutisch üblichen Hilfsstoffen, insbesondere Weichmachern formuliert sein kann,

Die multipartikuläre Arzneiform ist so formuliert, daß die enthaltenen Pellets im pH-Bereich des Magens freigesetzt werden.

Der Begriff Pellets im Sinne der Erfindung umfasst runde bis sphärische Agglomerate, die auch als Mikropartikel, Beads oder Minitabletten bezeichnet werden können, sofern sie den in der Erfindung beschriebenen Aufbau und Größe aufweisen.

Der äußere Überzug ist durch die Wahl des anionischen Polymeren bzw. Copolymeren bzw. seiner Formulierung mit Hilfsstoffen und seiner Schichtdicke so eingestellt, daß sich dieser in pH-Bereichen von 4,0 bis 8,0, bevorzugt von 5,5 bis 7,8, besonders bevorzugt 5,8 bis 7,5 im Darm innerhalb 15 bis 60, bevorzugt von 20 bis 40 min auflöst, so daß die wirkstoffhaltige, mucoadhaesive Matrix-Schicht frei wird, an die Darmmucosa binden und dort den Wirkstoff freisetzen kann.

Das Polymere bzw. Copolymere mit mucoadhaesiver Wirkung ist so gewählt, daß es in einem Bereich +/- 0,5, bevorzugt +/- 0,3 pH-Einheiten bezogen auf den pH-Wert, bei dem sich der äußere Überzug aufzulösen beginnt, eine mucoadhaesive Wirkung von mindestens η_{b} = 150 bis 1000, bevorzugt 150 bis 600 mPa·s und eine Wasseraufnahme von 10 bis 750, bevorzugt 10 bis 250, besonders bevorzugt 10 bis 160 % in 15 min aufweist und der Wirkstoffanteil der Matrixschicht maximal 40, insbesondere 0,001 bis 15 oder 0,05 bis 5 Gew.-% des Gehaltes am Polymeren mit mucoadhaesiver Wirkung beträgt.

### Die innere Matrix Schicht

Die innere Matrix-Schicht fungiert als Wirkstoffträger. Weiterhin hat die innere Matrix-Schicht die Funktion, den Wirkstoff mittels des enthaltenen mucoadhaesiven Polymeren an die Darmmucosa zu binden, so daß dieser von dort aus in den Organismus gelangen kann. Die innere Matrix Schicht hat weiterhin die Funktion den Wirkstoff vor physikalischer, chemischer oder enzymatischer Inaktivierung zu schützen.

Die innere Matrix kann zusätzlich pharmazeutische Hilfsstoffe, insbesondere G-Protein gekoppelte Rezeptoren und Liganden enthalten (s. z. B. WO 02/102407, S. 74-76), insbesondere 8-OH-DPAT, Aminoketanserin, Atropine, Butaclamol, Chlorpromazin, Chloroprozhixen, Cinanserin, Cyanopindolol, Cyproheptadin, Domperidon, Epi-depride, Epi-nephrine, Fenoldopam, Flupenthixol, Fluphenazin, Haloperidol, Hexocyclium, Himbacin, Iodomelatonin, Ketanserin, Lysergsäure-Derivate, Mesoridazine, Mesulerigin, Methoctramin, Methysergid, Metoclopramid, Mianserin, Molindonem, Muscarinic, Naloxon, N-Methylspiperon, Nor-epinephrin, Pergolid, Phentolamin, Pirenzepin, PPHT-Cumarin, PPHT-Rhodamin, PPHT-Texas red, Prazosin, Promazin, Racloprid, Serotonin, Speperone, Spriroxatrine, Sulpiride, Sumatriptan, Tenilapin und Trifluprimazin.

Desweiteren kann die innere Matrix Penetrationsförderer enthalten, z. B. Weichmacher wie beispielsweise Triethylcitrat, Acetyltrietylcitrat, Diethylsebacat, Dibutylsebacat, Polymere wie Carbomer, Chitosan, Chitosan-Cystein, Natrium-Carboxymethylcellulose, N-Trimethyliertes Chitosan, Polycarbophil-Cysteine, langkettige Fettsäuren, ihre Ester (beispielsweise Mono und Diglyceride) und ihre Salze wie Laurinsäure, Laurinsulfonsäure, Palmitinsäure, Caprylsäure, Caprinsäure, Ölsäure, Acylcarnitine, Chelatbildner wie EDTA, Salicylate, Cyclodextrine, Polyacrylsäuren, Gallensäuren wie Cholsäure, Cholyltaurin, Cholylsarcosin, Chenodeoxycholsäure und ihre Salze wie Na-Cholat, Na-Glykocholat, Na-Taurocholat, Na-Taurodihydrofusidat, Na-Glykodihydrofusidat, Tenside und Emulgatoren wie insbesondere Polyethylen-660-12-Hydroxy-Stearat (Solutol® HS15), (Solutol HS15), Polysorbat 80 (Tween 80), Polyoxyethyliertes Kastoröl (Cremophor EL), Polyoxyethylenepolyoxypropylene glycol (Pluronic® F68), das Toxin Zonula Occludens Toxin (ZOT) sowie Vitamine wie Vitamin E (Tocopherol) oder Vitamin B12.

Bevorzugt sind pharmazeutische Hilfsstoffe, Penetrationsförderer und/oder G-Protein gekoppelte Rezeptoren und Liganden in der inneren Matrix Schicht nicht oder nur in geringen Mengen, z. B. 0,01 bis 10, bevorzugt 0,05 bis 2, besonders bevorzugt 0,1 bis 1 Gew.-% enthalten.

### Nukleinsäure-Wirkstoffe

Die Matrix-Schicht enthält Nanopartikel mit einem Nukleinsäure-Wirkstoff. Der Nukleinsäure-Wirkstoff hat die Aufgabe am Zielort in-vivo eine Wechselwirkung mit der DNA von Säugerzellen, insbesondere menschlichen Zellen hervorzurufen, die zu einer veränderten DNA-Struktur in der Zelle oder ganz allgemein zu veränderten Zelleigenschaften führen. In erster Linie ist hier die sogenannte Gentherapie zu nennen, deren Ziel die Reparatur defekter Genstrukturen bei genetisch bedingten Erkrankungen ist. Hierbei kann es sich z. B. um eine Inaktivierung oder ein Abschalten unerwünschter Genaktivitäten handeln, wie z. B. der Telomeraseaktivität in Tumorzellen. Es kann sich auch um ein Wiederherstellen üblicherweise in gesunden Zellen vorhandener Genaktivitäten handeln, z. B. der p53-Genaktivität, einem seit lange bekannten, intensiv beforschten Tumorsuppressor-Gen. Die Erfindung betrifft demnach oral verabreichbare Arzneiformen für Nukleinsäure-Wirkstoffe, insbesondere für Gentherapie.

Der Nukleinsäure-Wirkstoff kann eine einzel- oder doppelsträngige DNA (Desoxyribonukleinsäure) oder RNA (Ribonukleinsäure) oder ein DNA-RNA-Chimär sein, wobei natürlich vorkommende und/oder nicht natürlich vorkommende synthetisch modifizierte Nukleotide enthalten sein können. Der Nukleinsäure-Wirkstoff kann linear oder ringförmig vorliegen. Es kann sich um Oligonukleotideinheiten, z. B. mit einer Länge von 10 bis 200 Basen oder Basenpaaren handeln. Es kann sich auch um längere Einheiten von z. B. über 200 bis 100.000, 500 bis 10.000 oder 1000 bis 5.000 Basen oder Basenpaaren handeln. Neben der als eigentlichem Wirkstoff fungierenden Sequenz, z. B. einer Nukleinsäuresequenz die in der Zielzelle vorhanden ist oder ergänzt werden soll, können im Nukleinsäure-Wirkstoff gegebenenfalls auch Vektor-sequenzen enthalten sein, die in der Regel nicht in der Zielzelle vorhanden sind und nicht mit dieser wechselwirken sollen.

Bekannt sind z. B. auf doppelsträngiger DNA basierende Vektorsysteme, die auf Plasmiden oder auf viralen Systemen basierenden Vektoren beruhen. Bekannt sind z. B. rekombinante Adeno-assozüerte-virale Vektoren (rAAV). Andere doppelsträngige Vektoren können Promotor- oder Regulationssequenzen aus Cytomegalieviren (CMV) oder dem SV40-Virus enthalten. Andere Vektoren können auf einzelsträngiger DNA beruhen, die mit Hilfe von angefügten RNA Elementen gegenüber Abbau geschützt werden kann. Bekannt sind auch sogenannte RDO I und RDO II Konstrukte, bei denen kurze DNA-Stücke, z. B. 30 bis 60 Basen an den Enden mit kurzen RNA Stücken von 1 bis 4 Basen versehen werden. Zur zusätzlichen Erhöhung der Halbwertszeit bzw. der Nukleaseresistenz können nicht natürlich vorkommende Nukleotide in die RNA oder DNA eingefügt werden. Dabei können z. B. einzelne Sauerstoffatome durch Schwefelatome ersetzt sein, so dass man Phosphor-Schwefel-Brücken erhält (MSO). Die Vielfalt als Gene-Repair oder Gene-Replacement-Vektoren geeigneten Nukleinsäure Formen, die als Wirkstoffe im Sinne der vorliegenden Erfindung eingesetzt werden können, ist z. B. Nature Reviews Vol.4, 2003, S. 679 - 689, Li Liu *et al.* beschrieben. Bevorzugt sind Nuklrinsäure-Fragmente, die im wesentlichen nur die als Wirkstoff fungierende Nukleinsäure-Sequenz und keine oder nur geringe Anteile an Vektor-DNA enthalten.

Der Nukleinsäure-Wirkstoff kann in einem Komplex oder Konjugat, z. B. mit kationischen Polymeren oder Proteinen wie z. B. Antikörpern, vorliegen. Die Komplexierung bzw. Konjugat-Bindung kann reversibel oder irreversibel kovalent durch chemische Brückenbindung oder nebenvalent über Van der Waals-Kräfte, ionische Bindungen, hydrophobe Bindung geschehen. Die neben dem Nukleinsäure-Wirkstoff im Komplex- oder Konjugat entfaltenen Moleküle entfalten jedoch selbst keine therapeutische Wirkung und sind somit als Formulierungshilfsmittel und nicht als Wirkstoff oder Teil des Wirkstoffs anzusehen.

Der Nukleinsäure-Wirkstoff kann gegebenenfalls unter Zuhilfenahme von Proteinen oder Peptiden formuliert werden. Diese entfalten jedoch selbst jedoch keine therapeutische Wirkung und sind somit als Formulierungshilfsmittel und nicht als Wirkstoff oder Teil des Wirkstoffs anzusehen.

Die Nukleinsäure kann z. B. gemäß der WO 02/094983 in Form eines Komplexes mit einem Antikörper, der spezifisch an die Nukleinsäure bindet, und einer kationischen Substanz vorliegt. Es konnte gezeigt werden, dass diese Maßnahme zu einer erhöhten Transfektionsrate sowohl in-vitro als auch in-vivo beitragen kann. Es kann sich dabei bevorzugt um monoklonale IgG-Antikörper oder IgM-Antikörper handeln, die vollständig oder auch als Fragmente, Fc-Antikörper-Fragmente, Fab'-Antikörper-Fragmente, F(a,b)'2-Antikörperfragmente oder halbe Antikörperfragmente handeln, die aber jeweils mindestens eine Anti-DNA-Bindungsstelle enthalten müssen. Das molekulare Verhältnis von Nukleinsäure zu Anti-DNA-Antikörper kann z. B. 1 zu 20 bis 1 zu 5 betragen.

Der Nukleinsäure-Wirkstoff kann z. B. die Therapie der Hämophilie zum Ziel haben und ein Blutgerinnungsfaktor-Gen, z. B. das cDNA-Gen des menschlichen Blutgerinnungsfaktors IX enthalten (s. z. B. WO 03/028657 oder Palmer et al., Blood, 1989, 73(2), p.438-445 oder Yao et al., Proc Natl Acad Sci USA, 1992, 89(8): p.3357-3361). Neben dem therapeutisch wirksamen Genanteil kann der Nukleinsäure-Wirkstoff auch ein Immuntoleranzinduzierendes Gen, wie z. B. den *Fas*-Ligand enthalten. Der coexprimierte *Fas-*Ligand bzw. -Genabschnitt kann die Apoptose in T-Zellen einleiten, die nach dem Gentransfer in die Zielzellen spezifisch aktiviert werden können. Vektoren in Zusammenhang mit der Apoptose-Induktion in Leukämie-Zellen sind auch aus Walensky et al., 2004, "Activation of Apoptosis in Vivo by a Hydrocarbon-Stapled BH3 Helix", Science, 305, S. 1466 -1470 abzuleiten.
Der Nukleinsäure-Wirkstoff kann z. B. einen Genabschnitt, insbesondere die Promotorregion, des humanen Telomerase-Gens enthalten. Geeignet ist z. B. der in WO 99/38964 beschriebene Gentherapie-Vektor pGT62-codAupp oder andere für einen Fachmann aus der WO 99/38964 ableitbare Vektoren. Der Nukleinsäure-Wirkstoff kann ein Tumorsuppressor-Genabschnitt enthalten, z. B. das p53-Tumorsuppressor-Gen oder Fragmente davon. US 6,451,593 B1 beschreibt Konstruktionsprinzipien für Expressionsvektoren für die Gentherapie, die zur Herstellung von Nukleinsäure-Wirkstoffen in Sinne der Erfindung geeignet sind.

### Nanopartikel

Die Arzneiform enthält Nanopartikel, die bevorzugt eine Größe im Bereich von 20 bis 1000, bevorzugt von 50 bis 250, besonders bevorzugt 80 bis 220, insbesondere von 100 bis 200 nm aufweisen können.

Die in den Nanopartikeln enthaltene Nukleinsäure kann bevorzugt in Form eines Komplexes mit einer kationischen Substanz vorliegt.

Die kationische Substanz kann ein kationisches Lipid, ein kationisches Polypeptid und/oder ein kationisches Polymer sein. Geeignet können auch Polyethylenimin oder Derivate sein.

Kationische Lipide können z. B. handelsübliche Mischungen aus N-[1-(2,3-dioleyloxy)propyl]-N-N-N-trimethylammonium-Chlorid (DOTMA) und Dioleylphosphatidylethanolamin (DOPE) sein. Geeignet sind z. B. auch N-[1-(2,3-dioleoyloxy)propyl]-N-N-N-trimethylammonium-methyl-sulfat (DOTAP), Dioleyl-phosphatidylcholine (DOPC), Dioctadecylamidoglycyl-spermin (DOGS).

Kationische Polypeptide sind bevorzugt synthetisch hergestellte Homopolymere von Aminosäuren mit kationischen Seitengruppen. Zu nennen sind Poly-Lysin, Poly-Arginin, Poly-Ornithin und Poly-Histidin. Die Kettenlängen können von wenigen Einheiten bis zu großen Einheiten variieren, z.B. 3 bis 20, 10 bis 50, 50 bis 100 oder bis 500 oder bis 1000 Aminosäuren. Es können auch natürlich vorkommende Proteine mit überwiegend kationischen Eigenschaften wie z. B. Histonproteine eingesetzt werden.

Bevorzugt gegenüber anderen Substanzen mit vergleichsweise geringerer pharmakologischer Erfahrung sind (Meth)acrylatcopolymere wegen ihrer seit Jahrzehnten sicheren Verwendung bei oral verabreichten Medikamenten.
Das kationische Polymer kann deshalb bevorzugt ein (Meth)acrylatcopolymer sein, insbesondere ein (Meth)acrylatcopolymer, welches tertiäre oder quaternäre Aminogruppen aufweist. Die Glastemperatur (ISO 11357-2, Punkt 3.3.3) des kationischen (Meth)acrylatcopolymers'liegt bevorzugt im Bereich von 40 bis 60 °C, das Molekulargewicht M_{w} (Gewichtsmittel) bei 100.000 bis 200.000 (Die Bestimmung des Molekulargewichts M_{w} kann beispielsweise per Gelpermeationschromatographie oder per Streulichtmethode erfolgen (siehe z. B. H. F. Mark et al., Encyclopedia of Polymer Science and Engineering, 2nd. Edition, Vol. 10, Seiten 1 ff., J. Wiley, 1989). Zur Verbesserung der Ausscheidung über die Niere oder den Gallenweg sind kationische (Meth)acrylatcopolymere mit niedrigem Molekulargewicht M_{w} bevorzugt, z. B. mit einem M_{w} von 50.000 oder weniger, 5.000 bis 40.000, 10.000 bis 30.000 oder 15.000 bis 25.000.

Das Molekulargewicht M_{w} (Gewichtsmittel) kann z. B. durch Viscosimetrie oder Gelauschlußchromatographie (GPC) bestimmt werden. Viscosimetrische Werte (Grenzviscositätszahl) können in Chloroform oder in DMF (Dimethylformamid) bei 23 °C bestimmt werden und sollen bevorzugt im Bereich von 10 bis 20, bevorzugt 11 bis 15 n_{spez/c} (cm³/g) liegen. Viscositätszahlen können z. B. nach ISO 1628-6 gemessen werden.

Besonders bevorzugt ist ein (Meth)acrylatcopolymer, das sich aus radikalisch polymerisierten Einheiten aus 20 - 30 Gew.-% Methylmethacrylat, 20 - 30 Gew.-% Butylmethacrylat und 60 - 40 Gew.-% Dimethylaminoethylmethacrylat zusammensetzt. Das (Meth)acrylatcopolymer kann insbesondere in mikronisierter Form mit mittleren Teilchengrößen von 10 bis 30 µm eingestezt werden. Ein konkret geeignetes handelsübliches (Meth)acrylatcopolymer mit tertiären Aminogruppen ist z. B. aus 25 Gew.-% Methylmethacrylat, 25 Gew.-% Butylmethacrylat und 50 Gew.-% Dimethylaminoethylmethacrylat aufgebaut (EUDRAGIT® E100). Besonders bevorzugt ist eine mikronisierte Form (EUDRAGIT® E PO, Pulver) mit einer mittleren Teilchengröße von 10 bis 20 µm. Diese lässt sich besonders gut zu Nukleinsäure-haltigen Nanopartikel verarbeiten. Dabei erhält man eine offenbar besonders günstige Komplexbildung mit den Nukleinsäure-Molekülen, die zu erhöhten Transfektionsraten beitragen kann.

### Nanopartikel, enthaltend Nukleinsäure-Wirkstoff sowie kationische und anionische (Meth)acrylatcopolymeren

Man kann die Transfektionsraten der jeweiligen Nukleinsäuren für den Zielzelltyp weiter optimieren, indem man bei der Herstellung der Nanopartikel, enthaltend Nukleinsäure-Wirkstoff und kationisches (Meth)acrylatcopolymer, zusätzlich ein anionisches (Meth)acrylatcopolymer in Anteilen von 0,1 bis 40, insbesondere 1 bis 30, besonders bevorzugt 2 bis 25 Gew.-%, bezogen auf den Nukleinsäure-Wirkstoff und das kationische (Meth)acrylatcopolymer, hinzugibt. Die Nanopartiklel müssen dann in einem in-vitro Assay mit einer Zellkultur des Zielzelltyps, sofern verfügbar, oder einem zumindest ähnlichen oder ähnlich reagierenden Zelltyp auf ihre Transfektionsrate hin geprüft werden. Auf diese Weise kann eine geeignete Balance zwischen den Bindekräften der Nukleinsäure im Komplex und ihrer Freisetzung aus dem Komplex in die lebende Zelle eingestellt werden. Ist die Bindewirkung durch das kationische (Meth)acrylatcopolymer allein anfangs zu stark, so dass die Transfektionsrate der Nukleinsäure unbefriedigend gering ist, kann die Bindungswirkung durch Zugabe des anionischen (Meth)acrylatcopofymers soweit abgeschwächt werden bis die Transfektionsrate ein Optimum erreicht, welches spezifisch für die eingesetzte Nukleinsäure und den Zielzelltyp ist. Diese Art der Formulierung hat den Vorteil, dass sowohl die kationischen als auch die anionischen (Meth)acrylatcopolymer pharmakologisch unbedenklich sind, so dass nicht oder nur kaum mit Nebenwirkungen zu rechnen ist.

Als anionische (Meth)acrylatcopolymere kommen bevorzugt die gleichen Typen in Betracht, die auch für den äußeren Überzug verwendet werden können, d.h. (Meth)acrylat-Copolymere mit einem Gehalt an Monomeren mit anionischen Gruppen von 5 bis 60 Gew.-% (EUDRAGIT^{®} Typen L, S, L100-55, FS). In vielen Fällen kann eine überraschende Erhöhung der Transfektionsraten erzielt werden, wenn man anionische (Meth)acrylatcopolymere einsetzt, aus
20 bis 33 Gew.-% Methacrylsäure und/oder Acrylsäure,
5 bis 30 Gew.-% Methylacrylat und
20 bis 40 Gew.-% Ethylacrylat und
größer 10 bis 30 Gew.-% Butylmethacrylat und
gegebenenfalls
0 bis 10 Gew.-% weiteren vinylisch copolymerisierbarer Monomeren,
wobei sich die Anteile der Monomeren zu 100 Gew.-% addieren,
mit der Maßgabe, daß die Glastemperatur des Copolymers (glass transition temperature) nach ISO 11357-2, Punkt 3.3.3 (midpoint temperature *T*_{mg}), 55 bis 70 °C beträgt.

Das oben genannte Copolymer setzt sich insbesondere zusammen aus radikalisch polymerisierten Einheiten von
20 bis 33, bevorzugt 25 bis 32, besonders bevorzugt 28 bis 31 Gew.-% Methacrylsäure oder Acrylsäure, bevorzugt ist Methacrylsäure,
5 bis 30, bevorzugt 10 bis 28, besonders bevorzugt 15 bis 25 Gew.-% Methylacrylat,
20 bis 40, bevorzugt 25 bis 35, besonders bevorzugt 18 bis 22 Gew.-% Ethylacrylat, sowie
größer 10 bis 30, bevorzugt 15 bis 25, besonders bevorzugt 18 bis 22 Gew.-% Butylmethacrylat
zusammen, wobei die Monomerzusammensetzung so gewählt wird, daß die Glastemperatur des Copolymers 55 bis 70 °C, bevorzugt 59 bis 66, besonders bevorzugt 60 bis 65 °C beträgt.

Zur Verbesserung der Ausscheidung über die Niere oder den Gallenweg sind anionische (Meth)acrylatcopolymere mit niedrigem Molekulargewicht bevorzugt, z. B. mit einem M_{w} von 50.000 oder weniger, 5.000 bis 40.000, 10.000 bis 30.000 oder 15.000 bis 25.000, bevorzugt.

Das Molekulargewicht M_{w} (Gewichtsmittel) kann z. B. durch Viscosimetrie oder Gelauschlußchromatographie (GPC) bestimmt werden. Viscosimetrische Werte (Grenzviscositätszahl) können in Chloroform oder in DMF (Dimethylformamid) bei 23 °C bestimmt werden und sollen bevorzugt im Bereich von 10 bis 20, bevorzugt 11 bis 15 n_{spez/c} (cm³/g) liegen. Viscositätszahlen können z. B. nach ISO 1628-6 gemessen werden.

Anionische (Meth)acrylatcopolymere mit niedrigem Molekulargewicht sind pHsensitive Polymere, die im Bereich von pH 7,0 oder leicht darüber keine oder nur in hohen Konzentrationen cytotoxische Eigenschaften aufweisen, aber unterhalb von pH 6,5 bereits in geringer Konzentration in vivo hämolytisch bzw. membranolytisch wirken. Die Polymere können in den Nanopartikeln als Modulatoren für die Bindungsstärke zwischen Nukleinsäure-Wirkstoff und kationischem (Meth)acrylatcopolymer dienen und zugleich die Transfektionsraten günstig beeinflussen. Der Anteil der anionischen (Meth)acrylatcopolymere mit niedrigem Molekulargewicht in den Nanopartikeln kann insbesondere dazu beitragen, dass der Nukleinsäure-Wirkstoff nach Aufnahme in Endosomen durch deren anschließende Destabilisierung oder Lyse intrazellulär freigesetzt wird.

### Anionische (Meth)acrylatcopolymere mit niedrigem Molekulargewicht für die Nano-Verkapselung

In einer bevorzugten Ausführungsform werden anionische (Meth)acrylatcopolymere mit niedrigem Molekulargewicht, z. B. mit einem M_{w} von 50.000 oder weniger, 5.000 bis 40.000, 10.000 bis 30.000 oder 15.000 bis 25.000, durch Nano-Verkapselung als Hülle auf Nanopartikel aufgebracht, die den Nukleinsäure-Wirkstoff und kationisches Polymer, bevorzugt ein kationisches (Meth)acrylatcopolymer enthalten. Der Anteil der anionischen (Meth)acrylatcopolymere mit niedrigem Molekulargewicht an der Oberfläche der Nanopartikeln kann insbesondere dazu beitragen, dass der Nukleinsäure-Wirkstoff nach Aufnahme in Endosomen durch deren anschließende - Destabilisierung oder Lyse intrazellulär freigesetzt wird. Zudem wird der Nukleinsäure-Wirkstoff im Inneren besser vor nukleolytischem Abbau geschützt, so dass mehr Wirkstoff zum Zielort gelangen kann.

### Wirkstoffanteile

Der Anteil der Nanopartikel in der Matrixschicht beträgt bevorzugt maximal 40, insbesondere 0,001 bis 15 oder 0,05 bis 5 Gew.-% des Gehaltes am Polymeren mit mucoadhaesiver Wirkung. In den Nanopartikeln kann der Anteil des Nukleinsäure-Wirkstoffs z. B. 1 bis 50, bevorzugt 2 bis 25 Gew.-% betragen.

### Herstellung von Nanopartikeln

Die Herstellung von Nanopartikeln ist bekannt. Bekannte Methoden sind Coacervation, Komplexbildung, Emulsions-Fällung, Verdampfen des organischen Lösungsmittelanteils aus einer Wasser-in-Öl-Emulsion, wodurch Nanopartikel in der wässrigen Phase entstehen. Verdampfen des organischen Lösungsmittelanteils aus einer Öl-in-Wasser-Emulsion, wodurch Nanopartikel in der wässrigen Phase entstehen. Leong et al. (1998) beschreibt die Herstellung von Nanopartikeln in Journal of Controlled Release 53, S. 183- 193 "DNA-polycation nanospheres as non-viral gene delivery vehicles*".* Roy et al. (1999) beschreibt die Herstellung von Nanopartikeln in Nature Medicine, Vol.5, Nr.4, S. 387 - 391, "Oral gene delivery with chitosan-DNA nanoparticles generates immunologic protection in murine model of peanut allergy*".*

Unter Nano-Verkapselung versteht man eine Grenzschichtpolymerisationsmethode (s. z.B. Chouinard F. et al., Pharm Res., 1994, Jun 111(6): 869-874). Nanokapseln können erzeugt werden, indem Nanopartikel als unlösliche Komplexe in wässrigem Medium dispergiert werden und die Dispersion in einem organischen Lösemittel emulgiert wird. Die Dispersion in einem organischen Lösemittel beinhaltet z. B. ein (Meth)acrylatcopolymer. Beim Verdampfen des organischen Lösemittels fällt das (Meth)acrylatcopolymer aus und bildet eine Hülle um die Nanopartikel.
Die Verkapselung der Nanopartikel ist vorteilhaft, weil ein zusätzlicher Schutz des komplexierten Nukleinsäure-Wirkstoffs während der Resorptionsprozesse durch die Enterocyten und die Leber gewährleistet wird.

### Polymere mit mucoadhaesiver Wirkung

Die Matrix-Schicht enthält weiterhin Polymere mit mucoadhaesiver Wirkung, Geeignete Polymere mit mucoadhaesiver Wirkung sind insbesondere ein Chitosan (Chitosan und Derivate, Chitosane), (Meth)acrylatcopolymere, bestehend aus 20 - 45 Gew.-% Methylmethacrylat und 55 bis 80 Gew.-% Methacrylsäure, Cellulosen, insbesondere Methylcellulosen, wie Na-Carboxymethylcellulose (z. B. Blanose® oder Methocel®). Bevorzugt gegenüber anderen Substanzen mit vergleichsweise geringerer pharmakologischer Erfahrung sind (Meth)acrylatcopolymere wegen ihrer seit Jahrzehnten sicheren Verwendung bei oral verabreichten Medikamenten.

Das Polymer mit mucoadhaesiver Wirkung ist so gewählt, daß es in einem Bereich +/- 0,5, bevorzugt +/- 0,3 pH-Einheiten bezogen auf den pH-Wert, bei dem sich der äußere Überzug aufzulösen beginnt, eine Wasseraufnahme von 10 bis 750, bevorzugt 10 bis 250, besonders bevorzugt 10 bis 160 % in 15 min aufweist.

### Messung der mucoadhäsiven Eigenschaften

Eine geeignete Messmethode zur Charakterisierung mucoadhäsiver Eigenschaften ist in *Hassan und Gallo* (1990) enthalten (s. Hassan E.E. und Gallo J. M. "A Simple Rheological Method for the in Vitro Assessment of Mucin-Polymer Bioadhaesive Bond Strength" Pharma Res. 7 (5), 491 (1990*)).* Die Methode beruht auf der Annahme, daß die Viscosität (η, dynamische Viscosität bzw. Viscositätskoeffizient) einer Mischung von Polymeren mit Mucin verschieden ist, von der Summe der Viskositäten der Einzelkomponenten. Es gilt der Zusammenhang η_{Mischung Polymer mit Mucin} = η_{Mucin} + η_{Polymer} + η_{b}, wobei η_{b} für die Differenz steht. Je höher η_{b}, desto höher sind die mucoadhaesiven Eigenschaften. Die einzelnen Komponenten werden zunächst mit einem Rotationsviscosimeter auf ihre Viscosität hin vermessen. Eingesetzt werden eine 0,5 %-ige (w/w) wässrige Lösung des mucoadhaesiven Polymeren und eine 15 %-ige Lösung von Mucin aus Schweinemagen. Zur Bestimmung der mucoadhaesiven Eigenschaften η_{b} werden Mucin und Polymer allein und in ihrer Mischung in den angegebenen Konzentrationen gemessen.

Das Polymer mit mucoadhaesiver Wirkung ist so gewählt, daß es in einem Bereich +/- 0,5, bevorzugt +/- 0,3 pH-Einheiten bezogen auf den pH-Wert, bei dem sich der äußere Überzug aufzulösen beginnt, eine mucoadhaesive Wirkung gemessen als Viskosität η_{b} von 150 bis 1000, bevorzugt 150 bis 600, mPa·s aufweist.

### Hydratisierung und Wasseraufnahme

Die Hydratisierung von Polymeren beruht auf der Affinität des Polymers Wasser aufzunehmen. Polymere schwellen durch diese Wasseraufnahme an. Dies liegt an einem Ungleichgewicht zwischen dem chemischen Potential des Wassers im Polymer und dem Wasser im umgebenden Medium. Das Wasser wird aufgrund des osmotischen Druckes des Polymers solange aufgenommen bis sich ein Gleichgewicht zwischen innerer und äußerer Phase eingestellt hat. Das Polymer ist dann zu 100% hydratisiert. Für Polymere mit niedrigem mittleren Molekulargewicht liegt dann eine Lösung vor. Für Polymere mit höherem Molekulargewicht oder vernetzten Polymeren entsteht ein Gel. Die Wasseraufnahme bis zur Gleichgewichtseinstellung kann z. B. bis zum 10 fachen des eigenen Gewichtes betragen entsprechend 1000% des Polymergewichtes.

### Messung der prozentualen Wasseraufnahme

Die Messung der prozentualen Wasseraufnahme ist dem Fachmann geläufig. Eine geeignete Methode ist z. B. im Lehrbuch der pharmazeutischen Technologie/Rudolf Voigt, Basel: Verlag Chemie, 5. völlig überarbeitete Auflage, 1984, S. 151, 7.7.6 unter "Aufsaugvermögen" beschrieben. Die Methode bedient sich der sogenannten Enslin-Apparatur, bei der eine Glasfilternutsche über einen Schlauch mit einer graduierte Pipette verbunden ist. Die Pipette ist genau horizontal so montiert, daß sie auf gleicher Höhe mit der Glasfritte liegt. Eine Wasseraufnahme von 100 % wird im vorliegenden Fall als eine Wasseraufnahme von 1 ml Wasser pro 1 g Polymeren mit mucoadhaesiver Wirkung in 15 min definiert.

Durch die vergleichsweise schnelle Wasseraufnahme bzw. Hydratisierung und den hohen Hydratisierungsgrad wird zu dem Zeitpunkt, ab dem sich der äußere Überzug aufzulösen beginnt, ein rascher Schutz des Wirkstoffs und eine unmittelbare Bindung an die Darmmucosa gewährleistet. Die Bindung des Wirkstoffs in der mucoadhaesiven Matrix soll nur gering sein, so daß dieser unmittelbar von der Darmmucosa in den Organismus übergehen kann.

### Steuerung des Matrix-pH-Wertes

Die mucoadhaesive Wirkung ist bei vielen mucoadhaesiven Polymeren pHabhängig. Der pH-Wert in der Matrix kann gezielt durch den Zusatz einer Säure, einer Base oder eines Puffersystems gesteuert werden. Die innere Matrix kann beispielsweise als Polymer mit mucoadhaesiver Wirkung ein Chitosan enthalten, das zusammen mit einem Acetat-Puffersystem eingesetzt wird. Der Acetat/Na-Acetat-Puffer, z. B. auf pH 5,0 bis 5,5 eingestellt, kann sich als Zusatzstoff in der Matrix befinden oder auf einem Kern auf dem die Matrix aufgetragen wird aufgebracht. Auf diese Weise kann Chitosan auch in Kombination mit verfilmten Überzügen eingesetzt werden, die sich bei höheren pH-Werten, z. B. pH 6,0 bis 8,0, aufzulösen beginnen. Trotz des hohen Umgebungs-pH-Wertes, bleibt der niedrige pH-Wert in der Mikroumgebung der Matrix erhalten. Man kann so die mucoadhaesiven Eigenschaften des Polymers in einem pH-Bereich nutzen, in dem es ansonsten nicht oder nicht in diesem Maße mucoadhaesiv wirken würde. Dies hat den Vorteil, daß man einen gewissen Schutz gegenüber Nukleasen erzielen kann, deren pH-Optimum in höheren pH-Bereichen liegt. Das gleiche Prinzip kann auch in umgekehrter Weise angewendet werden, indem man den pH-Wert der Matrix mittels des Zusatzes einer Base erhöht und mit einem verfilmten Überzug kombiniert, der sich bei niedrigeren pH-Werten auflöst.

### Beispiele zur Auswahl geeigneter mucoadhaesiver Polymere

Die Auswahl geeigneter mucoadhaesiver Polymere basiert auf ihren mucoadhaesiven Eigenschaften und ihrer Wasseraufnahmefähigkeit. Die Polymere sollen im jeweiligen pH-Bereich eine mucoadhaesive Wirkung von mindestens n_{b}= 150 bis 1000 mPa·s und eine Wasseraufnahme von 10 bis 750 % in 15 min aufweisen. Die folgende Tabelle gibt eine beispielhafte Auflistung.

Chitosan ist z. B. geeignet für die Anwendung in einem Umgebungs-pH-Bereich von pH 5,5 (Duodenum) oder bei einem anderen Umgebungs-pH-Bereich (Ileum oder Colon), sofern der Matrix-pH-Bereich, z. B. mit Hilfe eines Puffersystems, auf den Bereich um pH 5,5 eingestellt wurde.

Das in der Tabelle aufgelistete (Meth)acrylatcopolymer ist besser für einen Ph-Bereich von pH 7,2 als für einen pH-Bereich um pH 5,5 geeignet.

Na-Alginat ist für den pH-Bereich um pH 5,5 geeignet, jedoch nicht für pH 7,2.

Na-Carboxymethylcellulose und vernetzte Polyacrylsäure sind über einen weiten pH-Bereich von 5,5 bis 7,2 geeignet.

| Mucoadhesives Polymer | Mucoadhaesive Wirkung η_{b} [mPa·s] bei pH 5,5 | Mucoadhaesive Wirkung η_{b} [mPa·s] bei pH 7,2 | H₂O-Aufnahme [% in 15 min] bei pH 5,5 | H₂O-Aufnahme [% in 15 min] bei pH 6,0 | H₂O-Aufnahme [% in 15 min] bei pH 7,2 |
|---|---|---|---|---|---|
| Chitosan | 220 | 0 | 140 | 320 | 320 |
| (Meth)acrylatcopolymer * | 150 | 480 | 170 | 50 | 125 |
| Na-Alginat | 580 | 0 | 40 | 50 | 50 |
| Na-Carboxymethylcellulose | 300 | 250 | 55 | 50 | 50 |
| Polyacrylsäure vernetzt | 350 | 340 | 50 | 25 | 25 |

| | | | | | |
|---|---|---|---|---|---|
| *= (Meth)acrylatcopolymer aus 30 Gew.-% Methylmethacrylat und 70 Gew.% Methacrylsäure | | | | | |

### Der äußere Überzug aus anionischen (Meth)acrylatcopolymeren

Der äußere Überzug aus anionischen Polymeren oder Copolymeren dient als magensaftresistenter Überzug zum Schutz der inneren Matrixschicht vor den Magensäften. Weiterhin fungiert der äußere Überzug zum Schutz des Wirkstoffs vor nukleolytischen Enzymen bis zu dem Zeitpunkt, an dem sich der Überzug einen Darmabschnitt (Duodenum, Jejunum, Ileum oder Colon) erreicht, an dem er sich aufzulösen beginnt. Der äußere Überzug dient dabei insbesondere dem sogenannten "gastrointestinalen Targeting", d. h. der gezielten Freisetzung der inneren Matrixschicht, an den durch ihren dort herrschenden pH-Wert bestimmten Darmabschnitten. Damit es nicht zu einer Behinderung der Abgabe der inneren Matrix-Schicht kommt, soll das (Meth)acrylatcopolymere des äußeren Überzugs möglichst keine oder nur geringe Wechselwirkungen mit dem Wirkstoff oder dem mucoadhaesiven Polymeren der inneren Matrixschicht aufweisen.

Geeignete anionische Polymere bzw. Copolymere sind Celluloseglycolat (Duodcell®), Celluloseacetatphtalat (CAP, Cellulosi acetas, PhEur, Celluloseacetate-phtalate, NF, Aquateric®), Celluloseacetatsuccinat (CAS), Celluloseacetattrimeliat (CAT), Hydroxypropylmethylcellulosephtalat (HPMCP, HP50, HP55), Hydroxypropylmethylcelluloseacetatsuccinat (HPMCAS -LF, -MF, -HF), Polyvinylacetatphtalat (PVAP, Sureteric®), Vinylacetat-Vinylpyrolidon-Copolymer (PVAc, Kollidon® VA64), Vinylacetat:Crotonsäure-Copolymer 9:1 (VAC:CRA, Kollicoat® VAC) und oder Shelllack. Die genannten Polymere bzw. Copolymere lassen sich vielfach in durchaus befriedigender Weise so formulieren, daß eine pH-spezifische Auflösung erreicht werden kann.

Besonders bevorzugt besteht der äußere verfilmte Überzug im wesentlichen aus (Meth)acrylat-Copolymeren mit einem Gehalt an Monomeren mit anionischen Gruppen von 5 bis 60 Gew.-%, die optional mit pharmazeutisch üblichen Hilfsstoffen, insbesondere Weichmachern formuliert sein können. Gegenüber den eingangs genannten Polymeren bieten die genannten anionischen (Meth)acrylatcopolymere im Rahmen der Erfindung die Möglichkeit in vielen Fällen eine noch genauere und reproduzierbarere pH-spezifische Einstellung des Auflöse-pH-Wertes einzustellen. Auch die Handhabung und Applikation wird in der Regel als weniger aufwendig angesehen.

Das (Meth)acrylat-Copolymere für den äußeren Überzug besteht bevorzugt zu 40 bis 95, bevorzugt zu 45 bis 90, insbesondere zu 30 bis Gew.-% aus radikalisch polymerisierten C₁- bis C₄-Alkylestem der Acryl- oder der Methacrylsäure und kann 5 bis 60, bevorzugt 8 bis 40, insbesondere 20 bis 35 Gew.-% (Meth)acrylat-Monomere mit einer anionischen Gruppe enthalten.

In der Regel addieren sich die genannten Anteile zu 100 Gew.-%. Es können jedoch zusätzlich, ohne daß dies zu einer Beeinträchtigung oder Veränderung der wesentlichen Eigenschaften führt, geringe Mengen im Bereich von 0 bis 10, z. B. 1 bis 5 Gew.-% weiterer vinylisch copolymerisierbarer Monomere, wie z. B. Hydroxyethylmethacrylat oder Hydroxyethylacrylat enthalten sein.

C₁- bis C₄-Alkylestern der Acryl- oder Methacrylsäure sind insbesondere Methylmethacrylat, Ethylmethacrylat, Butylmethacrylat, Methylacrylat, Ethylacrylat und Butylacrylat.

Ein (Meth)acrylat-Monomer mit einer anionischen Gruppe kann z. B. Acrylsäure, bevorzugt jedoch Methacrylsäure sein.

Weiterhin geeignet sind anionische (Meth)acrylat Copolymere aus 40 bis 60, Gew.-% Methacrylsäure und 60 bis 40 Gew.-% Methylmethacrylat oder 60 bis 40 Gew.-% Ethylacrylat (Typen EUDRAGIT® L oder EUDRAGIT® L100-55). Die Glastemperatur (ISO 11357-2, Punkt 3.3.3) dieses Typs liegt im Bereich von 105 bis 160 °C, das Molekulargewicht M_{w} bei 100.000 bis 300.000 (Die Bestimmung des Molekulargewichts M_{w} kann beispielsweise per Gelpermeationschromatographie oder per Streulichtmethode erfolgen (siehe z. B. H. F. Mark et al., Encyclopedia of Polymer Science and Engineering, 2nd. Edition, Vol. 10, Seiten 1 ff., J. Wiley, 1989).

EUDRAGIT® L ist ein Copolymer aus 50 Gew.-% Methylmethacrylat und 50 Gew.-% Methacrylsäure. Dieses (Meth)acrylatcopolymer ist besonders geeignet zur Auflösung in pH-Bereichen um pH 6,0 bis 6,5 (Jejunum).

EUDRAGIT® L100-55 ist ein Copolymer aus 50 Gew.-% Ethylacrylat und 50 Gew.-% Methacrylsäure. EUDRAGIT® L 30D-55 ist eine Dispersion enthaltend 30 Gew.-% EUDRAGIT® L 100-55. Dieses (Meth)acrylatcopolymer ist besonders geeignet zur Auflösung in pH-Bereichen um pH 5,5 bis 6,0 (Duodenum)

Ebenso geeignet sind anionische (Meth)acrylat Copolymere aus 20 bis 40 Gew.-% Methacrylsäure und 80 bis 60 Gew.-% Methylmethacrylat (Typ EUDRAGIT® S). Dieses (Meth)acrylatcopolymer ist besonders geeignet zur Auflösung in pH-Bereichen um pH 6,5 bis 7,0 (Jejunum bzw. Ileum). Die Glastemperatur dieses Typs liegt im Bereich von 140 bis 180 °C, das Molekulargewicht M_{w} bei 100.000 bis 150.000.

Besonders gut geeignet sind (Meth)acrylat Copolymere, bestehend aus 10 bis 30 Gew.-%, Methylmethacrylat, 50 bis 70 Gew.-% Methylacrylat und 5 bis 15 Gew.-% Methacrylsäure.

EUDRAGIT® Typ FS ist ein Copolymer aus 25 Gew.-%, Methylmethacrylat, 65 Gew.-% Methylacrylat und 10 Gew.-% Methacrylsäure. EUDRAGIT® FS 30 D ist eine Dispersion enthaltend 30 Gew.-% des Copolymers Typ FS. Dieses (Meth)acrylatcopolymer ist besonders geeignet zur Auflösung in pH-Bereichen um pH 7,0 bis 7,8 (Ileum bzw. Colon)

Weiterhin geeignet ist ein Copolymer, welches sich aus
20 bis 34 Gew.-% Methacrylsäure und/oder Acrylsäure,
20 bis 69 Gew.-% Methylacrylat und
0 bis 40 Gew.-% Ethylacrylat und/oder gegebenenfalls
0 bis 10 Gew.-% weiteren vinylisch copolymerisierbarer Monomeren
zusammensetzt, mit der Maßgabe, daß die Glastemperatur des Copolymers nach ISO 11357-2, Punkt 3.3.3, höchstens 60 °C beträgt. Dieses (Meth)acrylatcopolymer ist wegen seiner guten Reißdehungseigenschaften insbesondere zum Verpressen von Pellets zu Tabletten geeignet.

Weiterhin geeignet sind Copolymere aus
20 bis 33 Gew.-% Methacrylsäure und/oder Acrylsäure,
5 bis 30 Gew.-% Methylacrylat und
20 bis 40 Gew.-% Ethylacrylat und
größer 10 bis 30 Gew.-% Butylmethacrylat und
gegebenenfalls
0 bis 10 Gew.-% weiteren vinylisch copolymerisierbarer Monomeren,
wobei sich die Anteile der Monomeren zu 100 Gew.-% addieren,
zusammensetzt, mit der Maßgabe, daß die Glastemperatur des Copolymers (glass transition temperature) nach ISO 11357-2, Punkt 3.3.3 (midpoint temperature T_{mg}), 55 bis 70 °C beträgt. Copolymere dieses Typs sind wegen seiner guten mechanischen Eigenschaften insbesondere zum Verpressen von Pellets zu Tabletten geeignet.

Das oben genannte Copolymer setzt sich insbesondere zusammen aus radikalisch polymerisierten Einheiten von
20 bis 33, bevorzugt 25 bis 32, besonders bevorzugt 28 bis 31 Gew.-% Methacrylsäure oder Acrylsäure, bevorzugt ist Methacrylsäure,
5 bis 30, bevorzugt 10 bis 28, besonders bevorzugt 15 bis 25 Gew.-% Methylacrylat,
20 bis 40, bevorzugt 25 bis 35, besonders bevorzugt 18 bis 22 Gew.-% Ethylacrylat, sowie
größer 10 bis 30, bevorzugt 15 bis 25, besonders bevorzugt 18 bis 22 Gew.-% Butylmethacrylat
zusammen, wobei die Monomerzusammensetzung so gewählt wird, daß die Glastemperatur des Copolymers 55 bis 70 °C, bevorzugt 59 bis 66, besonders bevorzugt 60 bis 65 °C beträgt.

Zur Einstellung spezieller Freisetzungsprofile bzw. Freisetzungsorte können auch Mischungen der genannten Copolymere zum Einsatz kommen.

Unter Glastemperatur wird hier insbesondere die midpoint temperature T_{mg} nach ISO 11357-2, Punkt 3.3.3, verstanden. Die Messung erfolgt ohne Weichmacherzusatz, bei Restmonomergehalten (REMO) von weniger als 100 ppm, bei einer Aufheizrate von 10 °C/min und unter Stickstoffatmosphäre.

Das Copolymer besteht bevorzugt in wesentlichen bis ausschließlich, zu 90, 95 oder 99 bis 100 Gew.-%, aus den Monomeren Methacrylsäure, Methylacrylat, Ethylacrylat und Butylmethacrylat in den oben angegebenen Mengenbereichen.

Es können jedoch zusätzlich, ohne daß dies zu einer Beeinträchtigung der wesentlichen Eigenschaften führen muß, geringe Mengen im Bereich von 0 bis 10, z. B. 1 bis 5 Gew.-% weiterer vinylisch copolymerisierbarer Monomere, wie z. B. Methylmethacrylat, Butylacrylat, Hydroxyethylmethacrylat, Vinylpyrrolidon, Vin,ylmalonsäure, Styrol, Vinylalkohol, Vinylacetat und/oder deren Derivate enthalten sein.

Die Copolymere werden in an sich bekannter Weise durch radikalische Substanz-, Lösungs-, Perl- oder Emulsionspolymerisation erhalten. Sie müssen vor der Verarbeitung durch geeignete Mahl-, Trocken- oder Sprühprozesse in den erfindungsgemäßen Teilchengrößenbereich gebracht werden.
Dies kann durch einfaches Brechen extrudierter und abgekühlter Granulatstränge oder Heißabschlag erfolgen.

Insbesondere bei Mischung mit weiteren Pulvern oder Flüssigkeiten kann der Einsatz von Pulvern vorteilhaft sein. Geeignete Gerätschaften zur Herstellung der Pulver sind dem Fachmann geläufig, z. B. Luftstrahlmühlen, Stiftmühlen, Fächermühlen. Gegebenenfalls können entsprechende Siebungsschritte einbezogen werden. Eine geeignete Mühle für industrielle Großmengen ist zum Beispiel eine Gegenstrahlmühle (Multi Nr. 4200), die mit ca. 6 bar Überdruck betrieben wird.

### Copolymerherstellung

Alle genannten (Meth)acrylatcopolymere sind durch radikalische Polymerisation der Monomeren in Gegenwart von Polymerisationsinitiatoren und Molekulargewichtsreglern mittels Block-, Perl- oder Emulsionspolymerisation und Austragen des Polymerisats erhältlich (siehe z. B. EP 0 704 207 A2, EP 0 704 208 A2 oder WO 03/092732). Die (Meth)acrylatcopolymere sind in an sich bekannter Weise durch radikalische Emulsionspolymerisation in wäßriger Phase in Gegenwart von vorzugsweise anionischen Emulgatoren herstellbar, beispielsweise nach dem in DE-C 2 135 073 beschriebenen Verfahren.
Als weitere Herstellungsverfahren sind grundsätzlich auch ,Group Transfer Polymerization' (GTP) oder ,Atom Transfer Radical Polymerization' (ATRP) geeignet (siehe z. B. Matyjaszewski, K. et al., Chem. Rev. 2001, 101, 2921-2990). Die resultierenden Polymerstrukturen sind statistische Copolymere oder Blockcopolymere.

Bevorzugt ist die Emulsionspolymerisation in Gegenwart von 2 bis 15 Gew.-% Molekulargewichtsreglern, einem Emulgatoranteil im Bereich von 0,1 bis 2 Gew.-%, einer Polymerisationinitiatormenge im Bereich von 0,02 bis 0,4 Gew.-% und bei Temperaturen von 65 bis 90 °C. Bevorzugt ist ein Emulgatorgemisch, bevorzugt aus Natriumlaurylsulfat, z. B. 0.1 bis 0,5 Gew.-%, und Polyoxyehtylen-20-Sorbitanmonooleat, z. B. 0,4 bis 1,5 Gew.-%. Als Initiatoren sind insbesondere Natriumperoxodisulfat oder Ammoniumperoxodisulfat geeignet. Man kann auf diese Weise z. B. eine Dispersion mit 20 bis 40 Gew.-% Feststoffgehalt herstellen und das Copolymer durch Sprühtrocknung oder durch Coagulation und Abpressen des Wasser in einem Extruder gewinnen. Anschließend wird das Polymerisat bevorzugt in einem organischen Lösemittel gelöst, durch mehrfache Dialyse gegen Wasser gereinigt und bevorzugt gefriergetrocknet.

Beispielhaft für Polymerisationsinitiatoren seien genannt: Azoverbindungen wie 2,2'-Azobis-(isobutyronitril) oder 2,2'-Azobis(2,4-dimethylvaleronitril), RedoxSysteme, wie beispielsweise die Kombination von tertiären Aminen mit Peroxiden oder bevorzugt Peroxide (vgl. hierzu beispielsweise H. Rauch-Puntigam, Th. Völker, "Acryl- und Methacrylverbindungen", Springer, Heidelberg, 1967 oder Kirk-Othmer, Encyclopedia of Chemical Technology, Vol. 1, Seiten 386ff, J. Wiley, New York, 1978). Beispiele geeigneter Peroxid-Polymerisationsinitiatoren sind Dilauroylperoxid, tert.-Butylperoctoat, tert.-Butylperisononanoat, Dicyclohexylperoxidicarbonat, Dibenzoylperoxid oder 2,2-Bis-(tert.-butylperoxy)-butan.

Man kann auch bevorzugt die Polymerisation mit einem Gemisch verschiedener Polymerisationsinitiatoren unterschiedlicher Halbwertzeit durchführen, beispielsweise Dilauroylperoxid und 2,2-Bis-(tert.-butylperoxy)-butan, um den Radikalstrom im Verlauf der Polymerisation sowie bei verschiedenen Polymerisationstemperaturen konstant zu halten. Die eingesetzten Mengen an Polymerisationsinitiator liegen im allgemeinen bei 0,01 bis höchstens 1 Gew.-% bezogen auf das Monomerengemisch.

Die Einstellung der Molekulargewichte M_{w} kann durch Polymerisation des Monomerengemisches in Gegenwart von Molekulargewichtsreglern erfolgen. Geeignete Molekulargewichtsregler sind insbesondere Mercaptane, wie beispielsweise n-Butylmercaptan, n-Dodecylmercaptan, 2-Mercaptoethanol oder 2-Ethylhexylthioglycolat, wobei die Molekulargewichtsregler im allgemeinen in Mengen von 0,05 bis 15 Gew.-% bezogen auf das Monomerengemisch, bevorzugt in Mengen von 0,1 bis 10 Gew.-% und besonders bevorzugt in Mengen von 2 bis 12 Gew.-% auf das Monomerengemisch eingesetzt werden (vgl. beispielsweise H. Rauch-Puntigam, Th. Völker, "Acryl- und Methacrylverbindungen", Springer, Heidelberg, 1967; Houben-Weyl, Methoden der organischen Chemie, Bd. XIV/1, Seite 66, Georg Thieme, Heidelberg, 1961 oder Kirk-Othmer, Encyclopedia of Chemical Technology, Vol. 1, Seiten 296ff, J. Wiley, New York, 1978). Bevorzugt wird als Molekulargewichtsregler n-Dodecylmercaptan oder 2-Ethylhexylthioglycolat eingesetzt. Ethylhexylthioglycolat bietet den Vorteil, daß die Hydrophobizität des (Meth)acrylat-Copolymeren beeinflußt werden kann, da der Regler endständig ins Molekül eingeht. 5 bis 15 Gew.-% Dodecylmercaptan oder 2 bis 10 Gew.-% 2-Ethylhexylthioglycolat sind bevorzugte Einsatzmengen.

### Organische Lösung

Die genannten (Meth)acrylatcopolymere können in Form einer organischen Lösung, z. B. in einer Konzentration von 10 bis 30 Gew.-%, bereitgestellt werden. Als Lösungsmittel können z. B. Aceton, Isopropanol oder Ethanol oder Mischung daraus verwendet werden, die gegebenenfalls Wasseranteile bis etwa 10 Gew.-% enthalten können. Bevorzugt sind jedoch wäßrige Dispersionen.

### Dispersionen

Die genannten (Meth)acrylatcopolymere können als Emulsionspolymerisate vorzugsweise in Form einer 10- bis 50-gew.-prozentigen, insbesondere 20 bis 40-prozentigen wäßrigen Dispersion erzeugt und angewendet werden. Als Handelsform ist ein Feststoffgehalt von 30 Gew.-% bevorzugt. Für die Verarbeitung ist eine teilweise Neutralisation der Methacrylsäure-Einheiten entbehrlich; sie ist jedoch, beispielsweise in einem Umfang bis zu 5 oder 10 Mol-% möglich, wenn eine Stabilisierung oder Verdickung der Überzugsmitteldispersion erwünscht sein sollte. Der Gewichtsmittelwert der Latex-Teilchengröße beträgt in der Regel 40 bis 100 nm, vorzugsweise 50 bis 70 nm, was eine verarbeitungstechnisch günstige Viskosität unter 1000 mPa·s gewährleistet.

Bei höheren Neutralisationsgrades z. B. 10 bis 50 Mol.-% oder vollständiger Neutralisation ist es möglich, das Copolymer in einen gelösten Zustand zu überführen.

Um eine Lösung des anionischen Copolymers herzustellen ist in der Regel eine teilweise oder vollständige Neutralisation der Säuregruppen nötig. Das anionische Copolymer kann z. B. nach und nach in einer Endkonzentration von 1 bis 40 Gew.-% in Wasser eingerührt werden und dabei teilweise oder vollständig neutralisiert werden durch Zugabe einer basischen Substanz wie z. B. NaOH, KOH, Ammoniumhydroxyd oder organische Basen wie z. B. Triethanolamin. Es ist auch möglich ein Pulver des Copolymeren einzusetzen, dem bereits bei seiner Herstellung zum Zweck der (Teil)neutralisation eine Base z. B. NaOH zugesetzt wurde, so daß das Pulver ein bereits (teil)neutralisiertes Polymer ist. Der pH-Wert der Lösung liegt in der Regel über 4, z. B. im Bereich von 4 bis ca. 7.

Die Dispersion kann z. B. auch in an sich bekannter Weise sprühgetrocknet oder gefriergetrocknet werden und im Form eines redispergierbaren Pulvers bereitgestellt werden (siehe z. B. EP-A 0 262 326). Alternative Verfahren sind die Gefriertrocknung oder Coagulation uns Abquetschen des Wassers in einem Extruder mit anschließender Granulation (siehe z. B. EP-A 0 683 028).

Überraschenderweise wurde gefunden, daß Copolymer-Dispersionen aus sprüh- oder gefriergetrockneten und redispergierten Pulvern eine erhöhte Scherstabilität aufweisen. Dies ist insbesondere beim Sprühauftrag von Vorteil. Dieser Vorteil tritt insbesondere verstärkt hervor wenn das in der Dispersion enthaltene Copolymer zu 2 bis 10 Mol-% in teilneutralisierter Form vorliegt (bezogen auf die im Copolymer enthaltenen Säuregruppen). Bevorzugt ist zu diesem Zweck die Teilneutalisation mittels Zugabe von NaOH. Bevorzugt ist ein anionischer Emulgator in Menge von 0,1 bis 2 Gew.-% enthalten. Besonders bevorzugt ist Natiumlaurylsulfat als Emulgator.

### Schichtdicken

Die Schichtdicke des äußeren Überzuges liegt bevorzugt im Bereich von 20 bis 200, bevorzugt von 50 bis 120 µm.

### Herstellung einer multipartikulären Arzneiform

Die Erfindung betrifft weiterhin ein Verfahren zur Herstellung einer multipartikulären Arzneiform, indem
a) einen Nukleinsäure-Wirkstoff mit Hilfsstoffen in an sich bekannter Weise zu Nanopartikeln formuliert wird,
b) eine innere Matrix-Schicht, enthaltend den Nukleinsäure-Wirkstoff in Form von Nanopartikeln und ein Polymer mit mucoadhaesiver Wirkung und gegebenenfalls weitere pharmazeutisch üblichen Hilfsstoffe mittels Sprühauftrag auf einen Kern oder durch Rotagglomeration, Ausfällen oder Sprühverfahren ohne einen Kern hergestellt wird und anschließend
c) ein äußerer verfilmter Überzug, bestehend im wesentlichen aus einem anionischen Polymeren, das optional mit pharmazeutisch üblichen Hilfsstoffen, insbesondere Weichmachern, formuliert sein kann, mittels Sprühauftag aufgebracht wird, so daß wirkstoffhaltige, umhüllte Pellets erhalten werden, und
d) die erhaltenen Pellets mittels pharmazeutisch üblicher Hilfsstoffe und in an sich bekannter Weise zu einer multipartikulären Arzneiform, insbesondere zu pellethaltigen Tabletten, Minitabletten, Kapseln, Sachets oder Trockensäften verarbeitet werden, die so formuliert sind, daß die enthaltenen Pellets im pH-Bereich des Magens freigesetzt werden.

### Herstellung von Prä-Pellets und Pellets

Die Pelletierung kann auf wirkstofffreie Kugeln (Nonpareilles) erfolgen oder es können kernfreie Pellets erzeugt werden.

Zunächst werden wirkstoffhaltige Nanopartikel erzeugt.

Anschließend wird die innere Matrixschicht mit oder ohne Kern erzeugt. Man kann diese noch nicht überzogene, ausgerundete Schicht als Prä-Pellet (Pelletkern) bezeichnen.

Mittels eines Wirbelschichtverfahrens kann eine Lösung oder Suspension des mucoadhaesiven Polymers, enthaltend die Nanopartikel mit dem Nukleinsäure-Wirkstoff, auf Placebopellets oder sonstige geeignete Trägermaterialien aufgebracht werden, wobei das Lösungs- oder Suspendiermittel verdunstet wird. Nach dem Herstellverfahren kann sich ein Trocknungsschritt anschließen.

Der Nukleinsäure-Wirkstoff wird in Form von Nanopartikeln mit dem Polymeren mit mucoadhaesiver Wirkung in einem organischen Lösemittel oder in Wasser eingetragen und vermischt. Um eine befriedigende Sprühbarkeit des Gemisches zu gewährleisten, ist es meist notwendig, ein Gemisch mit niedriger Viscosität zu formulieren. Zu diesem Zweck kann es günstig sein, das Polymere mit mucoadhaesiver Wirkung in vergleichsweise niedrigen Konzentrationen, z. B. von 1 bis höchstens 10, bevorzugt 2 bis 5 Gew.-% einzusetzen. Weiterhin kann der Zusatz eines Detergenz, z. B. Tween in Konzentrationen von 0,1 bis 20, bevorzugt 0,5 bis 10 Gew.-% zur Herabsetzung der Oberflächenspannung vorteilhaft sein.

Neben dem Wirkstoff können weitere pharmazeutische Hilfsstoffe enthalten sein: Bindemittel, wie Zellulose und deren Derivate, Polyvinylpyrrolidon (PVP), Feuchthaltemittel, Zerfallsförderer, Gleitmittel, Sprengmittel, (Meth)acrylate, Stärke und deren Derivate, Zucker, Solubilisatoren oder andere.

Entsprechende Auftragsverfahren sind z. B. aus Bauer, Lehmann, Osterwald, Rothgang, "Überzogene Arzneiformen" Wissenschaftliche Verlagsgesellschaft mbH Stuttgart, Kap 7, S.165 -196, bekannt.

Details sind dem Fachmann weiterhin aus Lehrbüchern bekannt. Siehe z. B.:
- Voigt, R. (1984): Lehrbuch der pharmazeutischen Technologie; Verlag Chemie Weinheim - Beerfield Beach/Florida - Basel.
- Sucker, H., Fuchs, P., Speiser, P. : Pharmazeutische Technologie, Georg Thieme Verlag Stuttgart (1991), insbesondere Kapitel 15 und 16, S. 626 - 642.
- Gennaro, A.,R. (Editor), Remington's Pharmaceutical Sciences, Mack Publishing Co., Easton Pennsylvania (1985), Chapter 88, S. 1567 - 1573.
- List, P. H. (1982): Arzneiformenlehre, Wissenschaftliche Verlagsgesellschaft mbH, Stuttgart.

Die innere Matrix kann auch ohne Zuhilfenahme eines inerten Kernes (Nonpareilles) erzeugt werden. Die Inhaltsstoffe der inneren Matrix können dabei durch Verfahren wie Rotagglomeration, Ausfällen oder Sprühverfahren, insbesondere Ultraschall-Wirbel-Sprühverfahren, zu noch nicht überzogenen Pellets (Prä-Pellets) definierter Größe, z. B. 50 bis 1000 µm, ausgerundet werden. Dies hat den Vorteil, daß das gesamte Kernvolumen zur Wirkstoffbeladung zur Verfügung steht. Die Wirkstoffbeladung kann dadurch gegenüber der Ausführungsform mit inertem Kern nochmals erhöht werden.

Nach Herstellung der inneren Matrix-Kerne (bzw. der Prä-Pellets) werden diese wiederum bevorzugt im Sprühverfahren mit dem äußeren Überzug versehen, so daß man fertige Pellets erhält. Die Herstellung der Pellets erfolgt mittels Sprühauftrag aus organischer Lösung, oder bevorzugt aus wäßrigen Dispersionen. Für die Ausführung ist dabei entscheidend, daß gleichmäßige, porenfreie Überzüge entstehen.

### Topcoat

Die Pellets können zusätzlich mit pigmentierten Überzügen versehen werden, die jedoch nicht den Auflöse-pH-Wert beeinflussen dürfen. Geeignet sind z. B. Überzüge aus pigmentierter Hydroxypropylmethylcellulose oder anderen wasserlöslichen bzw. in Wasser schnell zerfallenden Polymeren.

### Pharmazeutisch übliche Hilfsstoffe

Den erfindungsgemäßen Formulierungen können bei der Herstellung übliche Hilfs- bzw. Zuschlagstoffe hinzugefügt werden. Grundsätzlich müssen natürlich alle eingesetzten Substanzen toxikologisch unbedenklich und insbesondere in Arzneimitteln ohne Risiko für Patienten zu verwenden sein.

Einsatzmengen und Verwendung der üblichen Zuschlagstoffe in Arzneimittelüberzügen oder Beschichtungen sind dem Fachmann geläufig. Übliche Zuschlagstoffe können z. B. Weichmacher, Trennmittel, Pigmente, Stabilisatoren, Antioxidantien, Porenbildner, Penetrationsförderer, Glanzmittel, Aromastoffe, Detergenzien, Schmierstoffe oder Geschmacksmittel sein. Sie dienen als Verarbeitungshilfsmittel und sollen ein sicheres und reproduzierbares Herstellungsverfahren sowie gute Langzeitlagerstabilität gewährleisten oder sie erreichen in der Arzneiform zusätzliche vorteilhafte Eigenschaften. Sie werden den Polymerzubereitungen vor der Verarbeitung zugesetzt und können die Permeabilität der Überzüge beeinflussen, was ggf. als zusätzlicher Steuerparameter genutzt werden kann.

### • Trennmittel:

Trennmittel besitzen in der Regel lipophile Eigenschaften und werden in der Regel den Sprühsuspensionen zugesetzt. Sie verhindern eine Agglomeration der Kerne während der Befilmung. Bevorzugt werden Talkum, Mg- oder Ca - Stearat, gemahlene Kieselsäure, Kaolin oder nicht ionische Emulgatoren mit einem HLB - Wert zwischen 3 und 8 eingesetzt. Übliche Einsatzmengen für Trennmittel in den erfindungsgemäßen Überzugs- und Bindemitteln liegen zwischen 0,5 bis 100 Gew.-% bezogen auf das Copolymer.

### • Pigmente:

Mit dem Überzugsmittel unverträgliche Pigmente sind insbesondere solche Pigmente, die wenn sie der (Meth)acrylat-Copolymer-Dispersion direkt zugesetzt werden, z. B. durch Einrühren, in üblichen Anwendungsmengen von z. B. 20 bis 400 Gew.-% bezogen auf das Trockengewicht des (Meth)acrylat-Copolymeren zur Destabilisierung der Dispersion, Koagulation, zu Entmischungserscheinungen oder ähnlich unerwünschten Effekten führen. Weiterhin sind die zu verwendenden Pigmente natürlich nicht toxisch und für pharmazeutische Zwecke geeignet. Siehe dazu z. B. auch: Deutsche Forschungsgemeinschaft, Farbstoffe für Lebensmittel, Harald Boldt Verlag KG, Boppard (1978); Deutsche Lebensmittelrundschau 74, Nr. 4, S. 156 (1978); Arzneimittelfarbstoffverordnung AmFarbV vom 25.08.1980.

Mit dem Überzugsmittel unverträgliche Pigmente können z. B. Aluminiumoxidpigmente sein. Unverträgliche Pigmente sind z. B., Gelborange, Cochenillerotlack, Farbpigmente auf Basis von Aluminiumoxid bzw Azofarbstoffen, Sulfonsäurefarbstoffe, Gelborange S (E110, C.I. 15985, FD&C Yellow 6), Indigocarmin (E132, C.I. 73015, FD&C Blue 2), Tartrazin (E 102, C.I. 19140, FD&C Yellow 5), Ponceau 4R (E 125, C.I. 16255, FD&C Cochineal Red A), Chinolingleb (E 104, C.I. 47005, FD&C Yellow 10), Erythrosin (E127, C.I. 45430, FD&C Red 3), Azorubin (E 122, C.I. 14720, FD&C Carmoisine), Amaranth (E 123, C. I. 16185, FD&C Red 2), Brilliantsäuregrün (E 142, C.I. 44090, FD&C Green S).

Die angegebenen E-Nummern der Pigmente beziehen sich auf eine EU-Nummerierung. Siehe dazu auch "Deutsche Forschungsgemeinschaft, Farbstoffe für Lebensmittel, Harald Boldt Verlag KG, Boppard (1978); Deutsche Lebensmittelrundschau 74, Nr. 4, S. 156 (1978); Arzneimittelfarbstoffverordnung AmFarbV vom 25.08.1980. Die FD&C-Nummern beziehen sich auf die Zulassung in Food, Drugs und Cosmetics durch U.S. Food and Drug Administration (FDA) beschrieben in: U.S. Food and Drug Administration, Center for Food Safety and Applied Nutrition, Office of Cosmetics and Colors: Code of Federal Regulations - Title 21 Color Additive Regulations Part 82, Listing of Certified Provisionally Listed Colors and Specifications (CFR 21 Part 82).

### • Weichmacher

Weitere Zuschlagstoffe können auch Weichmacher sein. Übliche Mengen liegen zwischen 0 und 50, bevorzugt 2 bis 20, insbesondere 5 bis 10 Gew.-%.

Weichmacher können je nach Typ (lipophil oder hydrophil) und zugesetzter Menge die Funktionalität der Polymerschicht beeinflussen. Weichmacher erreichen durch physikalische Wechselwirkung mit dem Polymeren eine Absenkung der Glasübergangstemperatur und fördern in Abhängigkeit von der zugesetzten Menge die Verfilmung. Geeignete Stoffe haben in der Regel ein Molekulargewicht zwischen 100 und 20.000 und enthalten eine oder mehrere hydrophile Gruppen im Molekül, z. B. Hydroxyl-, Ester- oder Aminogruppen.

Beispiele geeigneter Weichmacher sind Citronensäurealkylester, Glycerinester, Phthalsäurealkylester, Sebacinsäurealkylester, Succroseester, Sorbitanester, Diethylsebacat, Dibutylsebacat und Polyethylenglykole 200 bis 12.000. Bevorzugte Weichmacher sind Triethylcitrat (TEC) und Acetyltriethylcitrat (ATEC). Weiterhin zu nennen sind in der Regel bei Raumtemperatur flüssige Ester wie Citrate, Phthalate, Sebacate oder Rizinusöl. Bevorzugt werden Zitronensäure- und Sebacinsäureester verwendet.

Die Zugabe der Weichmacher zur Formulierung kann in bekannter Weise, direkt, in wäßriger Lösung oder nach thermische Vorbehandlung der Mischung vorgenommen werden. Auch können Mischungen von Weichmachern eingesetzt werden

### Herstellung multipartikulärer Arzneiformen

Die wirkstoffhaltigen, überzogenen Pellets können mittels pharmazeutisch üblicher Hilfsstoffe und in an sich bekannter Weise zu multipartikulären Arzneiformen, insbesondere zu pellethaltigen Tabletten, Minitabletten, Kapseln, Sachets oder Trockensäften verarbeitet werden, die so formuliert sind, daß die enthaltenen Pellets im pH-Bereich des Magens freigesetzt werden. Die Darstellung als multipartikuläre Arzneiform stellt eine hohe Dosiersicherheit bietet den Vorteil einer guten Verteilung der Pellets im Darmlumen. Die erfindungsgemäße multipartikuläre Arzneiform kann zudem auch verschiedene Pellettypen mit unterschiedlichen Wirkstoffen und/oder unterschiedlichem Pellet-Aufbau enthalten.

### Verpresste Tabletten

Die Herstellung von multipartikulären Arzneiformen durch Verpressen eines pharmazeutisch üblichen Bindemittels mit wirkstoffhaltigen Partikeln ist z. B. Beckert et al. (1996), "Compression of enteric-coated pellets to disintegrating tablets", International Journal of Pharmaceutics 143, S. 13 - 23, und in WO 96/01624 beschrieben.

Filmüberzüge auf wirkstoffhaltige Pellets werden üblicherweise in Wirbelschichtgeräten aufgebracht. Filmbildner werden üblicherweise mit Weichmachern und Trennmitteln nach einem geeigneten Verfahren gemischt. Hierbei können die Filmbildner als Lösung oder Suspension vorliegen. Die Hilfsstoffe für die Filmbildung können ebenfalls gelöst oder suspendiert sein. Organische oder wässrige Löse- oder Dispergiermittel können verwendet werden. Zur Stabilisierung der Dispersion können zusätzlich Stabilisatoren verwendet werden (Beispiel: Tween 80 oder andere geeignete Emulgatoren bzw. Stabilisatoren).

Beispiele für Trennmittel sind Glycerolmonostearat oder andere geeignete Fettsäurederivate, Kieselsäurederivate oder Talkum. Beispiele für Weichmacher sind Propylenglykol, Phthalate, Polyethylenglykole, Sebacate oder Citrate, sowie andere in der Literatur erwähnte Substanzen.

Zwischen wirkstoffhaltiger und darmlöslicher Copolymer-Schicht kann eine trennende Schicht aufgebracht sein, die der Trennung von Wirkstoff und Überzugsmaterial zum Zwecke der Verhinderung von Interaktionen dient. Diese Schicht kann aus inerten Filmbildnern (z.B. HPMC, HPC oder (Meth)acrylsäure-Copolymeren) oder z.B. Talkum oder einer anderen geeigneten pharmazeutischen Substanzen bestehen. Ebenso können Kombinationen aus Filmbildnern und Talkum oder ähnlichen Stoffen verwendet werden.
Es ist auch möglich, eine Trennschicht aus teilweise bzw. vollneutralisierten (Meth)acrylatcopolymer-Dispersionen aufzubringen.

Die Trennschicht kann auch aus dem gleichen oder einem anderen mucoadhaesiven Polymer wie in der unterliegenden Matrixschicht bestehen. Auf diese Weise kann eventuellen Interaktionen oder Unverträglichkeiten des Wirkstoffs oder des mucoadhaesiven Polymers mit der filmbildenden (Meth)acrylatcopolymer-Schicht begegnet werden.

Mischungen zur Herstellung von Tabletten aus überzogenen Partikeln werden durch Vermischen der Pellets mit geeigneten Bindemitteln für die Tablettierung, nötigenfalls der Zugabe von zerfallsfördernden Substanzen und nötigenfalls der Zugabe von Schmiermitteln zubereitet. Das Mischen kann in geeigneten Maschinen stattfinden. Ungeeignet sind Mischer, die zu Schäden an den überzogenen Partikeln führen, z. B. Pflugscharmischer. Zur Erzielung geeigneter kurzer Zerfallszeiten kann eine spezielle Reihenfolge bei der Zugabe der Hilfsstoffe zu den überzogenen Partikel erforderlich sein. Durch Vormischung mit der überzogenen Partikel mit dem Schmier- oder Formentrennmittel Magnesiumstearat kann dessen Oberfläche hydrophobisiert und somit Verkleben vermieden werden.

Zum Tablettieren geeignete Mischungen enthalten üblicherweise 3 bis 15 Gew.-% eines Zerfallshilfsmittels, z. B. Kollidon CL und z. B. 0,1 bis 1 Gew.-% eines Schmier- und Formentrennmittels wie Magnesiumstearat. Der Bindemittelanteil bestimmt sich nach dem geforderten Anteil an überzogenen Partikeln. Typische Bindemittel sind z. B. Cellactose^{®}, mikrokristalline Cellulose, Calciumphosphate, Ludipress^{®}, Lactose oder andere geeignete Zucker, Calciumsulfate oder Stärkederivate. Bevorzugt werden Substanzen mit geringer Schüttdichte.

Typische Zerfallshilfsmittel (Sprengmittel) sind quervernetzte Stärke- oder Cellulosederivate, sowie quervernetztes Polyvinylpyrrolidon. Ebenso sind Cellulosederivate geeignet. Durch Auswahl eines geeigneten Bindemittels kann die Verwendung von Zerfallshilfsmittel entfallen.

Typische Schmier- und Formentrennmittel sind Magnesiumstearate oder andere geeignete Salze von Fettsäuren oder in der Literatur zu diesem Zweck aufgeführte Substanzen (z.B. Laurinsäure, Calciumstearat, Talkum usw.). Bei Verwendung geeigneter Maschinen (z.B. Tablettenpresse mit externer Schmierung) oder geeigneter Formulierungen kann die Verwendung eines Schmier- und Formentrennmittels in der Mischung entfallen.

Der Mischung kann gegebenenfalls ein Hilfsmittel zur Fließverbesserung beigefügt sein (z. B. hochdisperse Kieselsäurederivate, Talkum usw.).

Das Tablettieren kann auf üblichen Tablettenpressen, Exzenter- oder Rundlauftablettenpressen erfolgen, bei Preßkräften im Bereich von 5 bis 40 kN, bevorzugt 10 - 20 kN. Die Tablettenpressen können mit Systemen zur externen Schmierung ausgestattet sein. Gegebenenfalls kommen spezielle Systeme zur Matrizenbefüllung zum Einsatz, die die Matrizenbefüllung mittels Rührflügeln vermeiden.

### Weitere multipartuläre Arzneiformen

Alternativ zu verpressten Tabletten bzw. Minitabletten, können die wirkstoffhaltigen, überzogenenen Pellets auch zu beliebige anderen, oral verabreichbareb multipartikuläre Arzneiformen verarbeitet werden. Die überzogenen Pellets können z. B. in Kapseln, z. B. Gelatinekapsel, verfüllt werden oder zu Sachets oder Trockensäfte formuliert werden.

### Vorteilhafte Wirkungen der Erfindung

Die erfindungsgemäße Arzneiform eignet sich zur gezielten und effektiven Freisetzung von Nukleinsäure-Wirkstoffen. Die Arzneiform weist eine hohe Dosiersicherheit auf und verteilt sich gut im Magen und im Darmlumen. Der enthaltene Nukleinsäure-Wirkstoff wird dabei weitgehend gegenüber physikalischer oder nukleolytischer Inaktivierung geschützt und kann am definierten Wirkort so freigesetzt werden, daß ein hoher Anteil des Wirkstoffs von Körper aufgenommen werden kann. Die Arzneiform kommt daher mit weniger Wirkstoff aus, da nur wenig Wirkstoff verloren geht. Die Gefahr von Nebenwirkungen wird durch die gezielte Abgabe insgesamt verringert. Der Wirkort kann je nach therapeutischem Ziel variabel eingestellt werden. Der Zeitpunkt der Wirkstoffaufnahme kann somit besser gesteuert werden. Da es sich um eine orale Arzneiform handelt hat diese eine insgesamt bessere Akzeptanz der Patienten ("patient compliance") im Vergleich zu anderen Applikationsformen. Eine Vielzahl von Nukleinsäure-Wirkstoffen kann dadurch der peroralen Anwendung zugänglich gemacht werden. Die Applikationsrisiken sind oftmals geringer als insbesondere bei parenteralen Applikationen. Auch die Kosten der Applikation können gering gehalten werden, da kein Fachpersonal für die Applikation notwendig ist.

### Lipophile Matrix

Ein besonderer Aspekt der Erfindung ergibt sich wenn der Wirkstoff in Form von Nanopartikeln in eine lipophile Matrix eingebettet ist, die einen Schmelzpunkt oberhalb von 37 °C, bevorzugt oberhalb von 45 °C, besonders bevorzugt bevorzugt oberhalb von 55 °C aufweist und die wirkstoffhaltige lipophile Matrix in die Matrix aus dem Polymeren mit mucoadhaesiver Wirkung eingebettet ist. Die Formulierung in der lipophilen Matrix zielt darauf ab, die Löslichkeit bzw. die Bioverfügbarkeit des Wirkstoffs, bevorzugt von wenig oder schwerlöslichen Wirkstoffen (im Sinne der DAB 10, 2003) zu verbessern.

Unter einer lipophilen Matrix im Sinne der Erfindung versteht man eine Substanz oder eine Mischung von Substanzen, in der oder denen der Wirkstoff gelöst, suspendiert oder emulgiert werden kann. Die Substanz oder die Substanzen der lipophilen Matrix sind verschieden von den üblichen pharmazeutischen Hilfsstoffen und dem Polymer mit mucoadhaesiver Wirkung. Die Substanz oder die Substanzen der lipophilen Matrix haben bevorzugt einen hydrophoben oder auch amphiphilen Charakter. Man könnte die lipophile Matrix auch als amphiphile Matrix oder als lipoide Matrix bezeichnen.

Die lipophile Matrix kann aus einer einzelnen Substanz, z. B. einem Lipid, oder einer Mischung von Substanzen, z. B einer Mischung von Lipiden, bestehen. Im Falle von Mischungen errechnen sich die im folgenden beschriebenen Eigenschaften für Wasserlöslichkeiten nach DAB 10, Partitionskoeffizienten und/oder HLB-Werte jeweils aus dem arithmetrischen Mittel aus den Gewichtsteilen und den Werten der Substanzen der Mischung. Die eingesetzten Substanzen dürfen nicht toxisch sein.

### Lipophile Matrix/Polymere mit mucoadhaesiver Wirkung

In einer bevorzugt Ausführungsform werden mögliche Interaktionen der lipophile Matrix mit dem Polymer mit mucoadhaesiver Wirkung berücksichtigt. Um nicht kontrollierbare Wechselwirkungen zu vermeiden soll die Substanz oder die Substanzen, die die lipophile Matrix bilden, und das Polymere mit mucoadhaesiver Wirkung bevorzugt entweder die gleichen ionische Eigenschaften aufweisen, d.h. beide sollen übereinstimmend entweder zumindest überwiegend kationischen oder übereinstimmend anionischen Charakter haben. Im Falle, daß die Substanzen mit entgegengesetzten ionischer Eigenschaften ausgewählt werden, soll das Polymere mit mucoadhaesiver Wirkung bevorzugt zu mindestens 50, besonders bevorzugt zu 100 % in neutralisierter Form vorliegen. Die Neutralisation kann durch Zusatz von Säure oder Base in bekannter Weise erfolgen.

### Substanz oder Substanzen für den Aufbau der lipophilen Matrix

Bevorzugt besteht die lipophile Matrix zu 80 bis 100, bevorzugt zu 90 bis 100, besonders bevorzugt zu 100 Gew.-% aus einer Substanz oder einer Mischung von Substanzen mit einem (gemittelten) HLB-Wert von 0 bis 15, bevorzugt 2 bis 10 besteht. Die lipophile Matrix kann 0 bis 20, bevorzugt 0 bis 10 Gew.-% an pharmazeutisch üblichen Hilfsstoffen, insbesondere Stabilisatoren, Verdickungsmittel oder Adsorbentien, enthalten. Besonders bevorzugt sind keine pharmazeutisch üblichen Hilfsstoffe enthalten.

Die Substanz oder die Substanzen, die die lipophile Matrix bilden, können z. B. zu der Gruppe der Öle, Fette, Mono-, Di- oder Triglyceride, Fettsäuren, Fettalkohole, insbesondere C₆- bis C₂₀-Fettsäure und/oder einen C₆- bis C₂₀-Alkohol einschließlich deren Salze, Ether-, Ester- oder Amid-Derivaten, Phospholipide, Lecithine, Emulgatoren, Lipoide, lipidlösliches Vitamine oder Tenside gehören.

Die lipophile Matrix kann z. B. eine der folgenden Lipidpräparationen enthalten: (Imwitor 308) Glycerylmonocaprylate mit einem Monoesteranteil >80 %, (Imwitor 312) Glycerylmonolaurate mit enem Monoesteranteil > 90 %, (Imwitor 491) Glycerolmonosterate (C₁₆ + C₁₈) mit einem Monoesteranteil >90 %, (Imwitor 900 P) Glycerolmonosterat mit einem Monoesteranteil von 40 - 55% und einem C₁₈-Gehalt von 40 - 60%, (Imwitor 900 K) Glycerolmonosterat, mit einem Monoesteranteil von 40 - 55 % und einem C₁₈-Gehalt von 60 -80 %, (Imwitor 742) Mittelkettige C₈ und C₁₀ Glyceride mit einem Monoesteranteil von 45 - 55 %, (Imwitor 928) Partielle Glyceride gesättigter pflanzlicher C₁₀-C₁₈Fettsäuren mit einem Hauptanteil an C₁₂, und mit einem Monoesteranteil von 34-36%, C₈ and C₁₀-Glycedde, NaCaprylat oder NaCapriat.

Die lipophile Matrix kann z. B. eine der folgenden Lipidpräparationen enthalten: Fette wie Mono-, Di-, Triglyceride von gesättigten und ungesättigten Fettsäuren und deren Mischungen. Insbesondere Glycerin-Stearinsäureester, Glycerin-Palmitinsäureester, Glycerin-Myristinsäureester, Glycerin-Palmitinsäure-Stearinsäureester, Glycerin-Laurinsäureester Glycerin-Caprylsäureester, Glycerin-Ölsäureester, Beispiele für diese Ester sind Imwitor® - 308, - 312, - 491, 742, - 900, - 928, - 988, sowie Gelucire ® 44/14, - 50/13, Geleol, Compritol E ATO, Dynasan 114, Softisan, Witepsol, Dynacet 212, Kokosfett, Öle wie beispielsweise Rizinusöl, Sesamöl, Sonnenblumenöl, Baumwollsamenöl, Maisöl, Mandelöl, Erdnussöl, Olivenöl, Kokosnussöl, Karottenöl, Weizenkeimöl, Walnussöl, Neutralöle wie Isopropylmyristat, -palmitat, -stearat, mittelkettige Triglyceride (Miglyol^{®}).

Kurzkettige aliphatische und aromatische Carbonsäureester wie beispielsweise Dibutylphthalat, Diethylsebacat, Dibutylsebacat, Tributylcitrat, Acetyltributylcitrat, Glycerintriacetat,
Wachse wie beispielsweise Kanaubawachs, Bienenwachs, Wollwachs Glycerinbehensäureester,
Fettsäureamide wie beispielsweise Stearinsäureamid, Palmitinsäureamid, Laurinsäureamid,
Aliphatische langkettige Carbonsäuren wie beispielsweise Stearinsäure, Palmitinsäure, Laurinsäure, Myristinsäure, Ölsäure, Caprylsäure, Linolsäure, Linolensäure. Sowie beispielsweise deren Na-, AI- und Mg-Salze, Fettalkohole wie beispielsweise Stearylalkohol, Laurylalkohol, Cetylalkohol, Myristinalkohol, Glycerinformal,
W/O Emulgatoren wie beispielsweise Cholesterol, Glycerinmonostearat, Ethylenglycolmonostearat, Sorbitanmonooleat (Span® 80), -palmitat (Span® 40), -laurat (Span® 20), -stearat (Span® 60), Sorbitantrioleat (Span® 85)" Sorbitantristearat (Span®65), Sorbitansesquioleate (Arlacel® 83), Ca-, Al, Mg-Stearat, Polyoxethylensorbitantristearat (Tween® 65), Polyoxethylensorbitantrioleat (Tween® 85),
Nichtionische O/W Emulgatoren wie beispielsweise Macrogolstearat 400 (Chremophor® A), Macrogollaurylether, Polyethylenglykol-20-Sorbitanmonolaurat, -stearat, -palmitat, -oleat, Macrogol-1500-glycerintriricinoleat, Macrogolglycerinhydroxy-stearat (Cremophor® RH), Macrogol-1000-glycerinmono-laurat, -stearat, -oleat, Saccharosemonostearat. Polysorbat 60 (Tween® 60), Polyoxyethylenmonostearat (Myrj 49), Polysorbat 80 (Tween® 80), Polysorbat 40 (Tween® 40), Polysorbat 20 (Tween® 20), Poloxamer 407 (Lutrol® F 127), Poloxamer 188 (Lutrol® F 68), Polyoxyethlylenrizinoleat (Cremophor® EL), Polyoxyethylen-5-stearylstearat,
Ionische O/W Emulgatoren wie beispielsweise Cetylstearylsulfat (Lanette® E), Na-Laurylsulfat (Texapon® Z), Na-Glycocholat, Hederagenin,
Amphiphile Emulgatoren wie beispielsweise Ei-Phosphatidylcholin (Eilecithin), Soja-Phosphatidylcholin (Sojalecithin), Betain, Sulfobetaine, Ceramide (Sphingomyelin),
Vitamine wie beispielsweise Retinol (Vitamin A), Cholecalciferol (Vitamin D), alpha-Tocopherol und alpha-Tocopherolacetat (Vitamin E), Phyllochinon (Vitamin K),
Weitere Hilfsstoffe sind Galaktolipide wie beispielsweise Monogalaktosyldiacylglycerin, Digalaktosyl-diacylglycerin, Trigalaktosyl-diacylglycerin, sowie aromatische Öle wie beispielsweise Anisöl, Citronellöl, Eukalyptusöl, Fenchelöl, Kamillenöl, Kardamomenöl, Kiefernadelöl, Kümmelöl, Latschenöl, Lavendelöl, Minzöl, Muskatöl, Nelkenöl, Pfefferminzöl, Rosmarinöl, Salbeiöl
und Terpene wie beispielsweise Menthol, Linalool, 1,4-Cineol, Pyrethrin, Borneol, Eudesmol, Phytol, Manool, Azadirachtin, Nimbin.

Der Gehalt der wirkstoffhaltigen Lipidmatrix an der inneren Matrix-Schicht a) kann 1 bis 50, bevorzugt 10 bis 20 Gew.-% betragen.

Bevorzugt enthält die lipophile Matrix zu mindestens 50 Gew.-% Glycerinmonocaprylat, bis zu 10 Gew.-% Na-Cholat, bis zu 10 Gew.-% Tocopherol-Succinat, 1 bis 5 Gew.-% eines Efflux-Pumpen-Inhibitors im Falle, daß der Wirkstoff ein Substrat der PgP-Effluxpumpe ist, z. B. Solutol HS 15, ein Triglycerid, insbesondere Tristearat, wobei sich die Komponenten zu 100 % addieren. Diese lipophile Matrix kann direkt ins mucoadhaesive Polymer eingearbeitet werden oder in Wasser emulgiert in das mucoadhaesive Polymer eingearbeitet werden. Im letzteren Fall kann die Wasserphase eine schwache Säure, wie z. B. Citronensäure, beinhalten.

### Verfahren

Die Erfindung betrifft auch ein Verfahren zur Herstellung einer multipartikulären Arzneiform mit den Schritten
a) Erzeugen der wirkstoffhaltigen lipophilen Matrix durch Suspendieren der Nanopartikel, enthaltend den Nukleinsäure-Wirkstoff, mit der oder den Substanzen, die die lipophile Matrix bilden und gegebenenfalls weiteren pharmazeutisch üblichen Hilfsstoffen durch intensives Mischen oder Schmelzen der Inhaltsstoffe,
b) Erzeugen von Präpellets (Pelletkerne) mittels Sprühauftrag des mucoadhaesiven Polymers in Mischung mit der wirkstoffhaltigen lipophilen Matrix auf einen Kern oder durch Rotagglomeration, Ausfällen oder Sprühverfahren ohne einen Kern,
c) Erzeugen von Pellets durch Sprühauftrag eines Überzuges des anionischen Polymers oder Copolymers, das optional Beimengungen pharmazeutisch üblicher Hilfsstoffe, insbesondere Weichmachern und Trennmittel, enthalten kann, aus einer Dispersion oder organischer Lösung auf die Präpellets aus Schritt b),
d) Herstellung einer multipartikulären Arzneiform, durch Verfüllen oder Einarbeiten der Pellets aus Schritt c) in an sich bekannter Weise, gegebenenfalls unter Verwendung pharmazeutisch üblicher Hilfsstoffe, insbesondere durch Verarbeitung zu pellethaltigen Tabletten, Minitabletten, Kapseln, Sachets oder Trockensäften.

### Bevorzugtes Verfahren

Bevorzugt werden die Verfahrensschritte a) und b) wie folgt ausgeführt:
a) Erzeugen der inneren Matrix-Schicht durch Herstellen einer Emulsion oder Suspension der Nanopartikel, enthaltend den Nukleinsäure-Wirkstoff, mit der oder den Substanzen für die lipophile Matrix, und gegebenenfalls weiteren pharmazeutisch üblichen Hilfsstoffen durch intensives Mischen der Inhaltsstoffe in Wasser und Erzeugung einer Öl in Wasser-Präparation mit einer mittleren Teilchengröße von nicht mehr als 60, bevorzugt nicht mehr als 20 µm,
b) Erzeugen von Präpellets mittels Sprühauftrag der Öl in Wasser-Präparation aus Schritt a) auf das mucoadhaesive Polymer, das gegebenenfalls Beimengungen weiterer pharmazeutisch übliche Hilfsstoffe enthalten kann, wobei die Inhaltstoffe in Form eines mikronisierten Pulvers, z. B. mit einer mittleren Korngröße von 10 bis 100 µm, vorliegen, durch Rotagglomeration, Extrusion oder Granulation.

### BEISPIELE

Die Beispiele erläutern für die Erfindung typische Vorgehensweisen

### Beispiel 1

### Zubereitung von Nanopartikeln, enthaltend ein kationisches (Meth)acrylatcopolymer

2 mg DNA (Nukleinsäure-Wirkstoff), z. B. ein Gentherapievektor aus doppelsträngiger Plasmid-DNA mit z. B. 3000 bis 10.000 Basenpaaren, enthaltend ein in Humanzellen zu exprimierendes Gen mit beabsichtigter therapeutischer Wirkung, wird in 4ml Phosphatpuffer pH 7,4 gelöst und mit 2 ml einer Mouse-Monoclonal-Anti Human DNA IgM Lösung (1mg/ml) gemischt und eine Stunde lang bei 37°C inkubiert. Danach werden 1 ml Lipofectin™ oder bevorzugt 3 ml (1 mg/ml) modifiziertes EUDRAGIT® E ((Meth)acrylatcopolymer aus 25 Gew.-% Methylmethacrylat, 25 Gew.-% Butylmethacrylat und 50 Gew.-% Dimethylaminoethylmethacrylat, niedermolekular, nierengängig M_{w} = 21.000) gemischt und etwa 30 Minuten unter langsamen Rühren bei 37 °C behalten. Der pH-Wert wird nach dieser Zeit gemessen und mit HCl 0,001 N auf 7,4 justiert. Nach intensivem Mischen, z. B. auf einem Vortex, entstehen Nanopartikeln von etwa 250 nm mittlerem Durchmesser bei der Verwendung von Lipofectin® und von etwa 150 nm bei der Verwendung von modifiziertem EUDRAGIT® E. Die Suspension der Nanopartikel wird durch Dialyse gereinigt. Die Suspension kann direkt weiterverarbeitet werden oder die Nanopartikel können durch Gefriertrocknung separiert werden.

### Beispiel 2

### Nanopartikel, enthaltend kationisches und anionisches (Meth)acrylatcopolymer.

In Vorversuchen mit geeigneten Human-Zellkulturen wird ermittelt, dass eine optimale Transfektionsrate für den Nukleinsäure-Wirkstoff erreicht werden kann, wenn dem kationischen EUDRAGIT^{®} E (modifiziert) ein Anteil von z. B. 10 % des anionischen (Meth)acrylatcopolymers EUDRAGIT^{®} L (modifiziert) zugesetzt wird.

2 mg DNA (Nukleinsäure-Wirkstoff), z. B. ein Gentherapievektor aus doppelsträngiger Plasmid-DNA mit z. B. 3000 bis 10.000 Basenpaaren, enthaltend ein in Humanzellen zu exprimierendes Gen mit beabsichtigter therapeutischer Wirkung, wird in 4 ml Phosphatpuffer pH 7,4 gelöst und mit 2 ml einer Mouse-Monoclonal-Anti Human DNA IgM Lösung (1mg/ml) gemischt und eine Stunde lang bei 37°C inkubiert. Danach werden 1,1 ml, 4 ml (1mg/ml) modifiziertes EUDRAGIT® E ((Meth)acrylatcopolymer aus 25 Gew.-% Methylmethacrylat, 25 Gew.-% Butylmethacrylat und 50 Gew.-% Dimethylaminoethylmethacrylat, niedermolekular, nierengängig M_{w} = ca. 21.000) und 0,4 ml (1 mg/ml) modifiziertes EUDRAGIT® L (Copolymer aus 50 Gew.-% Methylmethacrylat und 50 Gew.-% Methacrylsäure, niedermolekular, nierengängig M_{w} = 21.000) gemischt und etwa 30 Minuten unter langsamen Rühren bei 37 °C behalten. Nach intensivem Mischen, z. B. auf einem Vortex, entstehen Nanopartikeln von etwa 250 nm mittlerem Durchmesser. Die Suspension der Nanopartikel wird durch Dialyse gereinigt. Die Suspension kann direkt weiterverarbeitet werden oder die Nanopartikel können durch Gefriertrocknung separiert werden.

### Beispiel 3

### Oberflächenmodifizierte Nanopartikel (Nanopartikel, enthaltend kationisches (Meth)acrylatcopolymer mit einer Hülle aus anionischem (Meth)acrylatcopolymer)

2 mg DNA (Nukleinsäure-Wirkstoff), z. B. ein Gentherapievektor aus doppelsträngiger Plasmid-DNA mit z. B. 3000 bis 10.000 Basenpaaren, enthaltend ein in Humanzellen zu exprimierendes Gen mit beabsichtigter therapeutischer Wirkung, wird in 4ml Dulbecco Phosphatpuffer pH 7,4 gelöst und mit 2 ml einer Mouse-Monoclonal-Anti Human DNA IgM Lösung (1mg/ml) gemischt und eine Stunde lang bei 37°C inkubiert. Danach werden 4 ml (1mg/ml) modifiziertes EUDRAGIT® E ((Meth)acrylatcopolymer aus 25 Gew.-% Methylmethacrylat, 25 Gew.-% Butylmethacrylat und 50 Gew.-% Dimethylaminoethylmethacrylat, niedermolekular, nierengängig M_{w}=21.000) gemischt und etwa 30 Minuten unter langsamen rühren bei 37 °C behalten. Der pH wird nach dieser Zeit gemessen und mit HCl 0,001 N auf 7.4 justiert.

Es werden 1 ml einer Lösung (1 mg/ml) aus modifiziertem EUDRAGIT® L ((Meth)acrylatcopolymer aus 50 Gew.-% Methylmethacrylat und 50 Gew.-% Methacrylsäure, niedermolekular, nierengängig, M_{w} = ca. 21.000) in PhosphatPuffer (pH 7.4, 0,5 mg/ml) dazugemischt und die resultierend latex-ähnliche Puffer-Dispersion wird unter Zugabe von eine 0,001 M Zitronensäure bis pH 5,0 erreicht wird. Die Suspension der Nanopartikel wird durch Dialyse gereinigt. Die Suspension kann direkt weiterverarbeitet werden oder die umhüllten Nanopartikel können durch Gefriertrocknung separiert werden.

### Beispiel 4

### Herstellung mucoadhaesiver noch nicht überzogener Pellets (Präpellets) durch Einbettung der Nanopartikeln aus den Beispielen 1, 2 oder 3 in eine innere Matrix-Schicht, die Chitosan enthält und mittels einer Säure auf pH 5,0 bis 5,5 eingestellt wird

Herstellung einer muco-adhaesiven Lösung:
4 g Chitosan-Acetat werden in 20 g Wasser aufgelöst. Unter schnellem Rühren werden danach 2 g Zitronensäure-Monohydrat zugegeben. Es wird ein pH von 5,2 eingestellt. In die erhaltene klare gelbliche viskose Lösung werden anschließend 0,4 g Na-Dodecanat zugesetzt. Zu dieser Lösung werden unter langsamen Rühren die Suspensionen aus Beispiel 1, 2 oder 3 gemischt.

### Herstellung der Präpellets

Die gemischte Suspension wird mit dem einem Wirbelschichtgerät (Micro-Lab von Hüttling) mit einer Sprührate von 5 - 8 g/min/kg auf 40 g Neutral-Pellets mit einen Durchmesser von ca. 400 - 600 µm bei einer Zulufttemperatur von 30 °C aufgesprüht. Die Zuluft wird hierbei auf 35 - 45 m³/h eingestellt. Die Ausbeute liegt hierbei bei 85 - 90 %.

### Beispiel 5

### Herstellung von (überzogenen) Pellets

Gemäß Beispiel 4 hergestellte Präpellets werden im Wirbelschichtverfahren mit EUDRAGIT® L12.5 ((Meth)acrylatcopolymer aus 50 Gew.-% Methylmethacrylat und 50 Gew.-% Methacrylsäure, M_{w} = ca. 200.000, 12,5 %-ige organische Lösung in Isopropanol/Aceton 3:2) überzogen. Der Polymerauftrag beträgt 40 Gew.-% bezogen auf das Kerngewicht. Die Suspension zum Überziehen besteht aus:

| | |
|---|---|
| EUDRAGIT® L12.5 | 53,3% |
| Triethylcitrat | 1,33% |
| Isopropanol | 38,3% |
| Talkum | 2,0% |
| Wasser | 5,0% |

Man erhält gleichmäßig umhüllte, magensaftresistente Pellets, deren Umhüllung sich oberhalb pH 6,0 in Duodenum oder Jejunum schnell auflöst und die mucoadhaesiven Präpellets freisetzt.

### Beispiel 6

### Herstellung von einer multipartikulären Arzneiform in Kapselform

Gemäß Beispiel 5 hergestellte Pellets werden mittels einer Kapselfüllvorrichtung in Hartgelatinekapseln, Kapseln der Grösse 0, unmittelbar zu Einheiten mit 550 mg Füllgewicht abgefüllt. Nach oraler Applikation löst sich die Kapsel im pH-Bereich des Magens schnell auf, setzt die Pellets frei, die sich bereits im Magen gleichmäßig verteilen.

### Beispiel 7

### Herstellung von einer multipartikulären Arzneiform in Tablettenform

Gemäß Beispiel 5 hergestellte Pellets werden mit Tablettierhilfsmitteln, Bindemitteln, Zerfallsförderer und Gleitmitteln formuliert. 550 g Pellets werden mit 390 g mikrokristalliner Cellulose, 150 g Na-Carboxymethylstärke und 10 g Mg-Stearat gemischt. Die Mischung wird in einer Tablettenpresse zu Presslingen mit einem Gesamtgewicht von 1100 mg verpresst. Nach oraler Applikation zerfällt die Tablette im pH-Bereich des Magens und setzt die Pellets frei, die sich bereits im Magen gleichmäßig verteilen.

## Patentansprüche

1. Orale multipartikuläre Arzneiform, enthaltend Pellets mit einem mittleren Durchmesser im Bereich von 50 bis 2500 µm, die im wesentlichen aufgebaut sind aus
a) einer inneren Matrix-Schicht, enthaltend Nanopartikel, die einen Nukleinsäure-Wirkstoff enthalten, und in eine Matrix aus einem Polymeren mit mucoadhaesiver Wirkung eingebettet sind, wobei die Matrix optional weitere pharmazeutisch übliche Hilfsstoffe enthalten kann,
b) einem äußeren verfilmten Überzug, bestehend im wesentlichen aus einem anionischen Polymeren oder Copolymeren, das optional mit pharmazeutisch üblichen Hilfsstoffen, insbesondere Weichmachern formuliert sein kann,
**dadurch gekennzeichnet, dass**
die multipartikuläre Arzneiform so formuliert ist, dass die enthaltenen Pellets im pH-Bereich des Magens freigesetzt werden, der äußere Überzug durch die Wahl des anionischen Polymeren oder Copolymeren bzw. seiner Formulierung mit Hilfsstoffen und seiner Schichtdicke so eingestellt ist, dass sich dieser in pH-Bereichen von 4,0 bis 8,0 im Darm innerhalb 15 bis 60 min auflöst, so dass die wirkstoffhaltige, mucoadhaesive Matrix-Schicht frei wird, an die Darmmucosa binden und dort den Wirkstoff freisetzen kann, wobei das Polymere mit mucoadhaesiver Wirkung so gewählt ist, dass es in einem Bereich +/-0,5 pH-Einheiten bezogen auf den pH-Wert, bei dem sich der äußere Überzug aufzulösen beginnt, eine mucoadhaesive Wirkung von mindestens η_{b}= 150 bis 1000 mPa·s und eine Wasseraufnahme von 10 bis 750 % in 15 min aufweist und der Wirkstoffanteil der Nanopartikel in der Matrixschicht maximal 40 Gew.-% des Gehaltes am Polymeren mit mucoadhaesiver Wirkung beträgt.

2. Arzneiform nach Anspruch 1, **dadurch gekennzeichnet, dass** die Nanopartikel eine Größe im Bereich von 20 bis 1000 nm aufweisen.

3. Arzneiform nach Anspruch 1 oder 2, **dadurch gekennzeichnet, dass** die in den Nanopartikeln enthaltene Nukleinsäure in Form eines Komplexes mit einer kationischen Substanz vorliegt.

4. Arzneiform nach Anspruch 3, **dadurch gekennzeichnet, dass** die kationische Substanz ein kationisches Lipid, ein kationisches Polypeptid und/oder ein kationisches Polymer ist.

5. Arzneiform nach Anspruch 4, **dadurch gekennzeichnet, dass** das kationische Polymer ein (Meth)acrylatcopolymer ist, welches tertiäre oder quaternäre Aminogruppen aufweist.

6. Arzneiform nach Anspruch 5, **dadurch gekennzeichnet, dass** sich das (Meth)acrylatcopolymer aus radikalisch polymerisierten Einheiten aus 20 - 30 Gew.-% Methylmethacrylat, 20 - 30 Gew.-% Butylmethacrylat und 60 - 40 Gew.-% Dimethylaminoethylmethacrylat zusammensetzt.

7. Arzneiform nach einem oder mehreren der Ansprüche 3 bis 6, **dadurch gekennzeichnet, dass** der in den Nanopartikeln enthaltene Nukleinsäure-Wirkstoff in Form eines Komplexes mit einem kationischen und einem anionischen (Meth)acrylatcopolymer vorliegt.

8. Arzneiform nach Anspruch 7, **dadurch gekennzeichnet, dass** das anionische (Meth)acrylat-Copolymere einen Gehalt an Monomeren mit anionischen Gruppen von 5 bis 60 Gew.-% aufweist.

9. Arzneiform nach Anspruch 7 oder 8, **dadurch gekennzeichnet, dass** ein anionisches (Meth)acrylatcopolymer enthalten ist, das sich aus
20 bis 33 Gew.-% Methacrylsäure und/oder Acrylsäure,
5 bis 30 Gew.-% Methylacrylat und
20 bis 40 Gew.-% Ethylacrylat und
größer 10 bis 30 Gew.-% Butylmethacrylat und gegebenenfalls
0 bis 10 Gew.-% weiteren vinylisch copolymerisierbarer Monomeren zusammensetzt, wobei sich die Anteile der Monomeren zu 100 Gew.-% addieren, mit der Maßgabe, daß die Glastemperatur des Copolymers (glass transition temperature) nach ISO 11357-2, Punkt 3.3.3 (midpoint temperature *T*_{mg}), 55 bis 70 °C beträgt.

10. Arzneiform nach einem oder mehreren der Ansprüche 3 bis 9, **dadurch gekennzeichnet, dass** die Nanopartikel kationische oder anionische (Meth)acrylatcopolymere mit einem mittleren Molekulargewicht M_{w} von 50.000 oder weniger enthalten.

11. Arzneiform nach einem oder mehreren der Ansprüche 1 bis 10, **dadurch gekennzeichnet, dass** die Nanopartikel eine Verkapselung mit einem anionischen (Meth)acrylatcopolymeren mit einem mittleren Molekulargewicht M_{w} von 50.000 oder weniger aufweisen.

12. Arzneiform nach einem oder mehreren der Ansprüche 1 bis 11, **dadurch gekennzeichnet, dass** der Nukleinsäure-Wirkstoff eine einzel- oder doppelsträngige DNA oder RNA oder ein DNA-RNA-Chimer ist, wobei natürlich vorkommende und/oder nicht natürlich vorkommende synthetisch modifizierte Nukleotide enthalten sein können.

13. Arzneiform nach einem oder mehreren der Ansprüche 1 bis 12, **dadurch gekennzeichnet, dass** die Nukleinsäure in Form eines Komplexes mit einem Antikörper, der spezifisch an die Nukleinsäure bindet, und einer kationischen Substanz vorliegt.

14. Arzneiform nach einem oder mehreren der Ansprüche 1 bis 13, **dadurch gekennzeichnet, dass** der äußere verfilmte Überzug Celluloseglycolat (Duodcell®), Celluloseacetatphtalat (CAP, Cellulosi acetas, PhEur, Celluloseacetate-phtalate, NF, Aquateric®), Celluloseacetatsuccinat (CAS), Celluloseacetattrimeliat (CAT), Hydroxypropylmethylcellulosephtalat (HPMCP, HP50, HP55), Hydroxypropylmethylcelluloseacetatsuccinat (HPMCAS -LF, -MF, -HF), Polyvinylacetatphtalat (PVAP, Sureteric®), Vinylacetat-Vinylpyrolidon-Copolymer (PVAc, Kollidon® VA64), Vinylacetat:Crotonsäure-Copolymer 9:1 (VAC:CRA, Kollicoat® VAC) und/oder Shelllack ist.

15. Arzneiform nach einem oder mehreren der Ansprüche 1 bis 13, **dadurch gekennzeichnet, dass** der äußere verfilmte Überzug aus einem (Meth)acrylat-Copolymeren mit einem Gehalt an Monomeren mit anionischen Gruppen von 5 bis 60 Gew.-% besteht.

16. Arzneiform nach einem oder mehreren der Ansprüche 1 bis 15, **dadurch gekennzeichnet, dass** die Schichtdicke des äußeren Überzuges im Bereich von 20 bis 200 *µ*m liegt.

17. Arzneiform nach einem oder mehreren der Ansprüche 1 bis 16, **dadurch gekennzeichnet, dass** die innere Matrix eine C₁₀- bis C₂₀-Fettsäure und/oder einen C₁₀- bis C₂₀-Alkohol einschließlich deren Salze, Ether-, Ester- oder Amid-Derivaten und/oder ein Lipid und/oder ein Phospholipid und/oder ein lipidlösliches Vitamin und/oder einen Penetrationsförderer enthält.

18. Arzneiform nach einem oder mehreren der Ansprüche 1 bis 17, **dadurch gekennzeichnet, dass** das Polymere mit mucoadhaesiver Wirkung ein Chitosan, ein (Meth)acrylatcopolymer, bestehend aus 20 - 40 Gew.-% Methylmethacrylat und 60 bis 80 Gew.-% Methacrylsäure und/oder eine Cellulose, insbesondere Na-Carboxymethylcellulose, eine vernetzte und/oder unvernetzte Polyacrylsäure, ein Lektin, ein Na-Alginat, und/oder ein Pektin ist.

19. Arzneiform nach Anspruch 18, **dadurch gekennzeichnet, dass** die innere Matrix als Polymer mit mucoadhaesiver Wirkung ein Chitosan enthält, das zusammen mit einer Säure oder einem Puffersystem eingesetzt wird, die bzw. das sich in der Matrix oder in oder auf einem Kern, auf dem die Matrix aufgetragen wird, befindet.

20. Arzneiform nach Anspruch 19, **dadurch gekennzeichnet, dass** die innere Matrix-Schicht Chitosan enthält und mittels einer Säure oder einem Puffersystem auf pH 5,0 bis 5,5 eingestellt wird und mit einem äußeren verfilmten Überzug kombiniert wird, der sich im Bereich von pH 6,0 bis 8,0 aufzulösen beginnt.

21. Arzneiform nach einem oder mehreren der Ansprüche 1 bis 20, **dadurch gekennzeichnet, dass** zwischen der wirkstoffhaltigen Matrix-Schicht und der äußeren verfilmten Überzugsschicht eine Trennschicht aufgebracht ist.

22. Verfahren zur Herstellung einer multipartikulären Arzneiform nach einem oder mehreren der Ansprüche 1 bis 21, durch die Schritte
a) Formulierung eines Nukleinsäure-Wirkstoffs mit Hilfsstoffen in an sich bekannter Weise zu Nanopartikeln,
b) Formulierung einer inneren Matrix-Schicht, enthaltend den Nukleinsäure-Wirkstoff in Form von Nanopartikeln und ein Polymer mit mucoadhaesiver Wirkung und gegebenenfalls weitere pharmazeutisch üblichen Hilfsstoffe mittels Sprühauftrag auf einen Kern oder durch Rotagglomeration, Ausfällen oder Sprühverfahren ohne einen Kern zu Präpellets und anschließend
c) Aufbringen eines äußeren verfilmten Überzugs mittels Sprühauftag auf die Präpellets, wobei der Überzug im wesentlichen aus einem anionischen Polymeren besteht, das optional mit pharmazeutisch üblichen Hilfsstoffen, insbesondere Weichmachern, formuliert sein kann, so daß wirkstoffhaltige, umhüllte Pellets erhalten werden, und
d) die erhaltenen Pellets mittels pharmazeutisch üblicher Hilfsstoffe und in an sich bekannter Weise zu einer multipartikulären Arzneiform, insbesondere zu pellethaltigen Tabletten, Minitabletten, Kapseln, Sachets oder Trockensäften verarbeitet werden, die so formuliert sind, daß die enthaltenen Pellets im pH-Bereich des Magens freigesetzt werden.

## Claims

1. Oral multiparticulate pharmaceutical form comprising pellets having an average diameter in the range from 50 to 2500 µm, which are composed essentially of
a) an inner matrix layer comprising nanoparticles which comprise a nucleic acid active ingredient, and are embedded into a matrix of a polymer having a mucoadhesive effect, where the matrix may optionally comprise further pharmaceutically usual excipients,
b) an outer film coating consisting essentially of an anionic polymer or copolymer which may optionally be formulated with pharmaceutically usual excipients, especially plasticizers,
**characterized in that**
the multiparticulate pharmaceutical form is formulated so that the contained pellets are released in the pH range of the stomach, the outer coating is adjusted through the choice of the anionic polymer or copolymer and its formulation with excipients and its layer thickness so that the coating dissolves in pH ranges from 4.0 to 8.0 in the intestine within 15 to 60 min so that the active ingredient-containing, mucoadhesive matrix layer is exposed and can bind to the intestinal mucosa and release the active ingredient there, where the polymer having a mucoadhesive effect is chosen so that it exhibits a mucoadhesive effect of at least η_{b} = 150 to 1000 mPa·s and a water uptake of from 10 to 750% in 15 min in a range of +/- 0.5 pH units relative to the pH at which the outer coating starts to dissolve, and the active ingredient content of the nanoparticles in the matrix layer is a maximum of 40% by weight of the content of polymers having a mucoadhesive effect.

2. Pharmaceutical form according to Claim 1, **characterized in that** the nanoparticles have a size in the range from 20 to 1000 nm.

3. Pharmaceutical form according to Claim 1 or 2, **characterized in that** the nucleic acid contained in the nanoparticles is present in the form of a complex with a cationic substance.

4. Pharmaceutical form according to Claim 3, **characterized in that** the cationic substance is a cationic lipid, a cationic polypeptide and/or a cationic polymer.

5. Pharmaceutical form according to Claim 4, **characterized in that** the cationic polymer is a (meth)acrylate copolymer which has tertiary or quaternary amino groups.

6. Pharmaceutical form according to Claim 5, **characterized in that** the (meth)acrylate copolymer is composed of free-radical polymerized units of 20-30% by weight methyl methacrylate, 20-30% by weight butyl methacrylate and 60-40% by weight dimethylaminoethyl methacrylate.

7. Pharmaceutical form according to one or more of Claims 3 to 6, **characterized in that** the nucleic acid active ingredient contained in the nanoparticles is present in the form of a complex with a cationic and an anionic (meth)acrylate copolymer.

8. Pharmaceutical form according to Claim 7, **characterized in that** the anionic (meth)acrylate copolymer has a content of monomers having anionic groups of from 5 to 60% by weight.

9. Pharmaceutical form according to Claim 7 or 8, **characterized in that** an anionic (meth)acrylate copolymer is present, which is composed of
20 to 33% by weight methacrylic acid and/or acrylic acid,
5 to 30% by weight methyl acrylate and
20 to 40% by weight ethyl acrylate and
more than 10 to 30% by weight butyl methacrylate and where appropriate
0 to 10% by weight further monomers capable of vinylic copolymerization, where the proportions of the monomers add up to 100% by weight, with the proviso that the glass transition temperature of the copolymer according to ISO 11357-2, subsection 3.3.3 (midpoint temperature *T_{mg}*) is from 55 to 70°C.

10. Pharmaceutical form according to one or more of Claims 3 to 9, **characterized in that** the nanoparticles comprise cationic or anionic (meth)acrylate copolymers having an average molecular weight M_{w} of 50 000 or less.

11. Pharmaceutical form according to one or more of Claims 1 to 10, **characterized in that** the nanoparticles have an encapsulation with an anionic (meth)acrylate copolymer having an average molecular weight M_{w} of 50 000 or less.

12. Pharmaceutical form according to one or more of Claims 1 to 11, **characterized in that** the nucleic acid active ingredient is a single- or double-stranded DNA or RNA or a DNA-RNA chimer, it being possible for naturally occurring and/or non-naturally occurring synthetically modified nucleotides to be present.

13. Pharmaceutical form according to one or more of Claims 1 to 12, **characterized in that** the nucleic acid is present in the form of a complex with an antibody which binds specifically to the nucleic acid, and of a cationic substance.

14. Pharmaceutical form according to one or more of Claims 1 to 13, **characterized in that** the outer film coating is cellulose glycolate (Duodcell®), cellulose acetate phthalate (CAP, Cellulosi acetas, PhEur, cellulose acetate phthalates, NF, Aquateric®), cellulose acetate succinate (CAS), cellulose acetate trimeliate (CAT), hydroxypropyl-methylcellulose phthalate (HPMCP, HP50, HP55), hydroxypropylmethylcellulose acetate succinate (HPMCAS -LF, -MF, -HF), polyvinyl acetate phthalate (PVAP, Sureteric®), vinyl acetatevinylpyrrolidone copolymer (PVAc, Kollidon® VA64), vinyl acetate:crotonic acid 9:1 copolymer (VAC:CRA, Kollicoat® VAC) and/or shellac.

15. Pharmaceutical form according to one or more of Claims 1 to 13, **characterized in that** the outer film coating consists of a (meth)acrylate copolymer having a content of monomers having anionic groups of from 5 to 60% by weight.

16. Pharmaceutical form according to one or more of Claims 1 to 15, **characterized in that** the layer thickness of the outer coating is in the range from 20 to 200 µm.

17. Pharmaceutical form according to one or more of Claims 1 to 16, **characterized in that** the inner matrix comprises a C₁₀ to C₂₀ fatty acid and/or a C₁₀ to C₂₀ alcohol including their salts, ether, ester or amide derivatives and/or a lipid and/or a phospholipid and/or a lipid-soluble vitamin and/or a penetration promoter.

18. Pharmaceutical form according to one or more of Claims 1 to 17, **characterized in that** the polymer having a mucoadhesive effect is a chitosan, a (meth)acrylate copolymer consisting of 20-40% by weight methyl methacrylate and 60 to 80% by weight methacrylic acid and/or a cellulose, in particular Na carboxymethylcellulose, a crosslinked and/or uncrosslinked polyacrylic acid, a lectin, an Na alginate, and/or a pectin.

19. Pharmaceutical form according to Claim 18, **characterized in that** the inner matrix comprises as polymer having a mucoadhesive effect a chitosan which is employed together with an acid or a buffer system which is present in the matrix or in or on a core onto which the matrix is applied.

20. Pharmaceutical form according to Claim 19, **characterized in that** the inner matrix layer comprises chitosan and is adjusted to pH 5.0 to 5.5 with an acid or a buffer system, and is combined with an outer film coating which starts to dissolve in the region of pH 6.0 to 8.0.

21. Pharmaceutical form according to one or more of Claims 1 to 20, **characterized in that** a separating layer is applied between the active ingredient-containing matrix layer and the outer film coating layer.

22. Process for producing a multiparticulate pharmaceutical form according to one or more of Claims 1 to 21, by the steps
a) formulating a nucleic acid active ingredient with excipients in a manner known per se to nanoparticles,
b) formulating an inner matrix layer comprising the nucleic acid active ingredient in the form of nanoparticles and a polymer having a mucoadhesive effect and, where appropriate, further pharmaceutically usual excipients by means of spray application onto a core or by rotagglomeration, precipitation or spray processes without a core to form pre-pellets and subsequently
c) applying an outer film coating consisting essentially of an anionic polymer, which may optionally be formulated with pharmaceutically usual excipients, especially plasticizers, by spray application to the pre-pellets so that active ingredient-containing enveloped pellets are obtained, and
d) processing the resulting pellets by means of pharmaceutically usual excipients and in a manner known per se to a multiparticulate pharmaceutical form, in particular to pellet-containing tablets, minitablets, capsules, sachets or powders for reconstitution, which are formulated so that the contained pellets are released in the pH range of the stomach.

## Revendications

1. Forme galénique multiparticulaire orale, contenant des granules ayant un diamètre moyen dans la plage de 50 à 2 500 µm, qui sont essentiellement constitués de
a) une couche-matrice interne, contenant des nanoparticules qui contiennent une substance active de type acide nucléique et sont incluses dans une matrice à base d'un polymère à activité muco-adhésive, la matrice pouvant en option contenir d'autres adjuvants pharmaceutiquement usuels,
b) un enrobage pelliculé externe, constitué essentiellement d'un polymère ou copolymère anionique qui peut en option être formulé avec des adjuvants pharmaceutiquement usuels, en particulier des plastifiants,
**caractérisée en ce que**
la forme galénique multiparticulaire est formulée de manière que les granules contenus soient libérés dans l'intervalle de pH de l'estomac, l'enrobage externe est ajusté par le choix du polymère ou copolymère anionique ou de sa formulation avec des adjuvants et de son épaisseur de couche, de manière qu'il se dissolve dans des intervalles de pH de 4,0 à 8,0 dans l'intestin en l'espace de 15 à 60 min, de sorte que la couche-matrice muco-adhésive contenant une substance active est libérée, peut se fixer à la muqueuse intestinale et y libérer la substance active, le polymère à activité muco-adhésive étant choisi de manière que dans un intervalle de +/- 0,5 unité de pH, par rapport au pH auquel l'enrobage externe commence à se dissoudre, il présente une activité muco-adhésive d' au moins η_{b} = 150 à 1 000 mPa.s et une absorption d'eau de 10 à 750 % en 15 min et la teneur en substance active des nanoparticules dans la couche-matrice représente au maximum 40 % en poids de la teneur en polymère à activité muco-adhésive.

2. Forme galénique selon la revendication 1, **caractérisée en ce que** les nanoparticules ont une taille dans la plage de 20 à 1 000 nm.

3. Forme galénique selon la revendication 1 ou 2, **caractérisée en ce que** l'acide nucléique contenu dans les nanoparticules est présent sous forme d'un complexe avec une substance cationique.

4. Forme galénique selon la revendication 3, **caractérisée en ce que** la substance cationique est un lipide cationique, un polypeptide cationique et/ou un polymère cationique.

5. Forme galénique selon la revendication 4, **caractérisée en ce que** le polymère cationique est un copolymère de (méth)acrylate qui comporte des groupes amino tertiaires ou quaternaires.

6. Forme galénique selon la revendication 5, **caractérisée en ce que** le copolymère de (méth)acrylate est composé de motifs polymérisés par voie radicalaire de 20 - 30 % en poids de méthacrylate de méthyle, 20 - 30 % en poids de méthacrylate de butyle et 60 - 40 % en poids de méthacrylate de diméthylaminoéthyle.

7. Forme galénique selon une ou plusieurs des revendications 3 à 6, **caractérisée en ce que** la substance active de type acide nucléique contenue dans les nanoparticules se trouve sous forme d'un complexe avec un copolymère de (méth)acrylate cationique et un copolymère de (méth)acrylate anionique.

8. Forme galénique selon la revendication 7, **caractérisée en ce que** le copolymère de (méth)acrylate anionique présente une teneur en monomères à groupes anioniques de 5 à 60 % en poids.

9. Forme galénique selon la revendication 7 ou 8, **caractérisée en ce qu'**est contenu un copolymère de (méth)acrylate anionique qui est composé de
20 à 33 % en poids d' acide méthacrylique et/ou d'acide acrylique,
5 à 30 % en poids d'acrylate de méthyle et
20 à 40 % en poids d'acrylate d'éthyle et
plus de 10 à 30 % en poids de méthacrylate de butyle et éventuellement
0 à 10 % en poids d'autres monomères copolymérisables avec des composés vinyliques, la somme des proportions des monomères étant égale à 100 % en poids, étant entendu que la température de transition vitreuse du copolymère (glass transition temperature) selon ISO 11357-2, Point 3.3.3 (température du point médian *T*_{*m*g}) vaut de 55 à 70 °C.

10. Forme galénique selon une ou plusieurs des revendications 3 à 9, **caractérisée en ce que** les nanoparticules contiennent des copolymères de (méth)acrylate cationiques ou anioniques ayant une masse moléculaire moyenne M_{w} de 50 000 ou moins.

11. Forme galénique selon une ou plusieurs des revendications 1 à 10, **caractérisée en ce que** les nanoparticules présentent une encapsulation avec un copolymère de (méth)acrylate anionique ayant une masse moléculaire moyenne M_{w} de 50 000 ou moins.

12. Forme galénique selon une ou plusieurs des revendications 1 à 11, **caractérisée en ce que** la substance active de type acide nucléique est un ADN ou ARN simple brin ou double brin ou une chimère ADN-ARN, dans lequel/laquelle peuvent être contenus des nucléotides existant dans la nature et/ou des nucléotides n'existant pas dans la nature, modifiés par synthèse.

13. Forme galénique selon une ou plusieurs des revendications 1 à 12, **caractérisée en ce que** l'acide nucléique est présent sous forme d'un complexe avec un anticorps qui se lie spécifiquement à l'acide nucléique, et avec une substance cationique.

14. Forme galénique selon une ou plusieurs des revendications 1 à 13, **caractérisée en ce que** l'enrobage pelliculé externe consiste en glycolate de cellulose, (Duodcell®), acétate-phtalate de cellulose (CAP, Cellulosi acetas, PhEur, acétate-phtalate de cellulose, NF, Aquateric®), acétate-succinate de cellulose (CAS), acétate-trimellitate de cellulose (CAT), phtalate d'hydroxypropylméthylcellulose (HPMCP, HP50, HP55), acétate-succinate d'hydroxypropylméthylcellulose (HPMCAS -LF, -MF, -HF), poly (acétate-phtalate de vinyle) (PVAP, Sureteric®), copolymère acétate de vinyle-vinylpyrrolidone (PVAc, Kollidon® VA64), copolymère acétate de vinyle/acide crotonique 9:1 (VAC:CRA, Kollicoat® VAC) et/ou gomme-laque.

15. Forme galénique selon une ou plusieurs des revendications 1 à 13, **caractérisée en ce que** l'enrobage pelliculé externe est constitué d'un copolymère de (méth)acrylate ayant une teneur en monomères à groupes anioniques de 5 à 60 % en poids.

16. Forme galénique selon une ou plusieurs des revendications 1 à 15, **caractérisée en ce que** l'épaisseur de couche de l'enrobage externe se situe dans la plage de 20 à 200 µm.

17. Forme galénique selon une ou plusieurs des revendications 1 à 16, **caractérisée en ce que** la matrice interne contient un acide gras en C₁₀ à C₂₀ et/ou un alcool en C₁₀ à C₂₀, y compris leurs sels, dérivés éthers, esters ou amides et/ou un lipide et/ou un phospholipide et/ou une vitamine soluble dans les lipides et/ou un agent favorisant la pénétration.

18. Forme galénique selon une ou plusieurs des revendications 1 à 17, **caractérisée en ce que** le polymère à activité muco-adhésive est un chitosane, un copolymère de (méth)acrylate, constitué de 20 - 40 % en poids de méthacrylate de méthyle et 60 à 80 % en poids d'acide méthacrylique et/ou une cellulose, en particulier la carboxyméthylcellulose sodique, un poly(acide acrylique) réticulé et/ou non réticulé, une lectine, un alginate de Na et/ou une pectine.

19. Forme galénique selon la revendication 18, **caractérisée en ce que** la matrice interne contient en tant que polymère à activité muco-adhésive un chitosane, qui est utilisé conjointement avec un acide ou un système tampon, qui se trouve dans la matrice ou est appliqué dans ou sur un noyau sur lequel est appliquée la matrice.

20. Forme galénique selon la revendication 19, **caractérisée en ce que** la couche-matrice interne contient du chitosane et est ajustée à pH 5, 0 à 5, 5 au moyen d'un acide ou d'un système tampon et est associée à un enrobage pelliculé externe qui commence à se dissoudre dans l'intervalle de pH 6,0 à 8,0.

21. Forme galénique selon une ou plusieurs des revendications 1 à 20, **caractérisée en ce qu'**entre la couche-matrice contenant une substance active et la couche d'enrobage pelliculé externe est appliquée une couche de séparation.

22. Procédé pour la fabrication d'une forme galénique multiparticulaire selon une ou plusieurs des revendications 1 à 21, par les étapes
a) formulation d'une substance active de type acide nucléique avec des adjuvants, d'une façon connue en soi, en nanoparticules,
b) formulation d'une couche-matrice interne, contenant la substance active de type acide nucléique sous forme de nanoparticules et un polymère à activité muco-adhésive et éventuellement d'autres adjuvants pharmaceutiquement usuels, par application par pulvérisation sur un noyau ou par agglomération rotative, précipitation ou un procédé de pulvérisation sans un noyau, en pré-granules et ensuite
c) application d'un enrobage pelliculé externe par application par pulvérisation sur les pré-granules, l'enrobage consistant essentiellement en un polymère anionique qui peut en option être formulé avec des adjuvants pharmaceutiquement usuels, en particulier des plastifiants, de sorte qu'on obtient des granules enrobés contenant une substance active, et
d) on transforme les granules obtenus, au moyen d'adjuvants pharmaceutiquement usuels et d'une façon connue en soi, en une forme galénique multiparticulaire, en particulier en comprimés, minicomprimés, gélules, sachets ou sirops secs, contenant des granules, qui sont formulés de telle façon que les granules contenus sont libérés dans l'intervalle de pH de l'estomac.
